(19) Europäisches Patentamt
European Patent Office
Office européen des brevets

(11) EP 4 400 494 A1

(12) EUROPEAN PATENT APPLICATION
published in accordance with Art. 153(4) EPC

(43) Date of publication:
**17.07.2024 Bulletin 2024/29**

(21) Application number: **22867724.1**

(22) Date of filing: **08.09.2022**

(51) International Patent Classification (IPC):
***C07D 295/145*** (2006.01) ***A61K 31/495*** (2006.01)
***A61K 31/496*** (2006.01) ***A61K 31/444*** (2006.01)
***A61P 25/00*** (2006.01) ***A61P 25/28*** (2006.01)
***C07D 307/68*** (2006.01) ***C07D 333/38*** (2006.01)
***C07D 213/81*** (2006.01) ***A61P 25/18*** (2006.01)

(52) Cooperative Patent Classification (CPC):
**A61K 31/444; A61K 31/495; A61K 31/496; A61P 25/00; A61P 25/18; A61P 25/28; C07D 213/81; C07D 295/145; C07D 307/68; C07D 333/38**

(86) International application number:
**PCT/KR2022/013519**

(87) International publication number:
**WO 2023/038456 (16.03.2023 Gazette 2023/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.09.2021 KR 20210119526**

(71) Applicant: **Neuroventi Inc.**
**Seoul 05029 (KR)**

(72) Inventors:
- **SHIN, Chan Young**
  **Seoul 07987 (KR)**
- **KWON, Kyoung Ja**
  **Seoul 05096 (KR)**
- **REMONDE, Chilly Gay**
  **Malaybalay City, Bukidnon, 8700 (PH)**
- **JEON, Se Jin**
  **Seoul 03009 (KR)**
- **CHO, Kyu Suk**
  **Seoul 07987 (KR)**
- **CHOI, Jung Hyun**
  **Seoul 05118 (KR)**
- **GONZALES, Edson Luck**
  **Maasin City, Southern Leyte, 6600 (PH)**
- **SONG, Eun Yeong**
  **Seoul 05101 (KR)**
- **JOO, So Hyun**
  **Seoul 01619 (KR)**
- **PAUDEL, Suresh**
  **Tanahun, 33900 (NP)**
- **KIM, Kyeong Man**
  **Gwangju 61037 (KR)**
- **CHEONG, Jae Hoon**
  **Jeonju-si, Jeollabuk-do 54957 (KR)**

(74) Representative: **Stolmár & Partner Patentanwälte PartG mbB**
**Blumenstraße 17**
**80331 München (DE)**

# (54) NOVEL COMPOUND AND USE THEREOF FOR TREATING EMOTIONAL BEHAVIORAL DISORDERS

(57) The present invention relates to a novel compound, and a use thereof for treating emotional and behavioral disorders. The pharmaceutical composition for preventing or treating emotional and behavioral disorder, which includes the novel composition of the present invention inhibits agmatinase and is expected to be helpful for improving autism spectrum disorder, emotional and behavioral disorder such as schizophrenia, as well as emotional and behavioral disorder-related symptoms such as lack of sociality, hyperactivity or the like.

EP 4 400 494 A1

## Description

[Technical Field]

The present invention relates to a novel compound, and a use thereof for treating emotional and behavioral disorders.

[Background Art]

Emotional and behavioral disorders are the only mental disorders for which there is no cure, and it may be defined as a spectrum disease that exhibits difficulties in social and communication and stereotyped behaviors, and shows various coexisting symptoms such as seizures, anxiety, depression, aggression, cognitive impairment and the like.
The clear pathogenesis of emotional and behavioral disorders has not been identified, and the subject on which the most research has been conducted is the excitation/inhibition imbalance. By regulating the imbalance of excitatory/inhibitory neurons, it influences neural connectivity and integration, suggesting the possibility of improving core symptoms of emotional and behavioral disorders such as autism.

[Summary of Invention]

[Problems to be Solved by Invention]

An object of the present invention is to provide a novel compound, a stereoisomer or a pharmaceutically acceptable salt thereof.
Another object of the present invention is to provide a novel compound useful for the prevention or treatment of emotional and behavioral disorders, a stereoisomer or a pharmaceutically acceptable salt thereof.
In addition, another object of the present invention is to provide a novel compound, a stereoisomer or a pharmaceutically acceptable salt thereof, which is useful for preventing or treating emotional and behavioral disorder-related symptoms.
Further, another object of the present invention is to provide a pharmaceutical composition for the prevention or treatment of emotional and behavioral disorders, which includes a novel compound, a stereoisomer or a pharmaceutically acceptable salt thereof.

[Means for Solving Problems]

1. A compound represented by Formula 1 below, a stereoisomer or a pharmaceutically acceptable salt thereof:

[Formula 1]

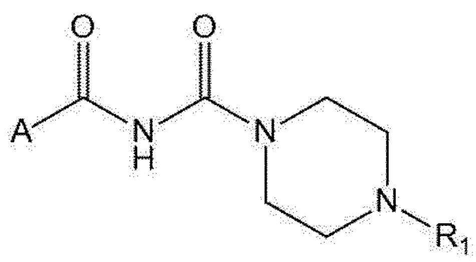

(wherein, $R_1$ is hydrogen or a tert-butyloxycarbonyl group,
A is a single or double cyclic group of $C_5$-$C_{10}$,
each ring of the cyclic group is unsubstituted or substituted with 1 to 3 heteroatoms, and
the cyclic group is unsubstituted or substituted with halogen, $C_1$-$C_5$ alkyl, $C_1$- $C_5$ alkoxy, CN or $NO_2$).

2. The compound, a stereoisomer or a pharmaceutically acceptable salt thereof according to the above 1, wherein A is selected from the group consisting of the following cyclic groups:

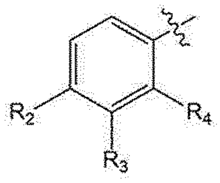 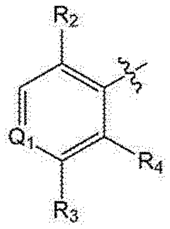 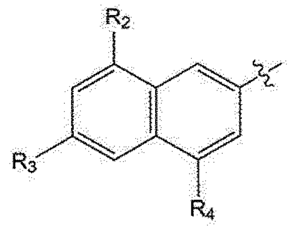

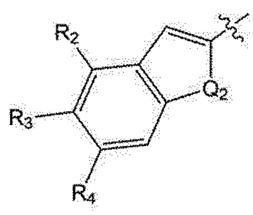 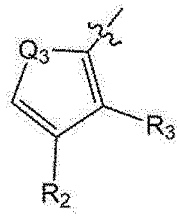

(wherein $R_2$, $R_3$ and $R_4$ are each independently hydrogen, halogen, $C_1$-$C_5$ alkyl, $C_1$-$C_5$ alkoxy, CN or $NO_2$, and $Q_1$, $Q_2$ and $Q_3$ are each independently N, O or S).

3. The compound, a stereoisomer or a pharmaceutically acceptable salt thereof according to the above 1, wherein A is selected from the group consisting of the following cyclic groups:

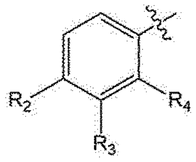 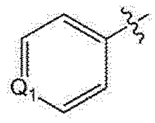 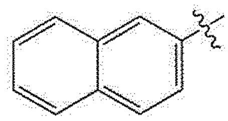

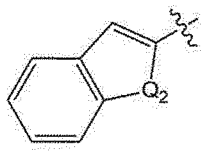 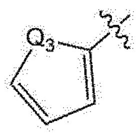

(wherein $R_2$, $R_3$ and $R_4$ are each independently hydrogen, halogen, $C_1$-$C_5$ alkyl, $C_1$-$C_5$ alkoxy, CN or $NO_2$; and $Q_1$, $Q_2$ and $Q_3$ are each independently N, O or S).

4. The compound, a stereoisomer or a pharmaceutically acceptable salt thereof according to the above 1, wherein A is selected from the group consisting of the following cyclic groups:

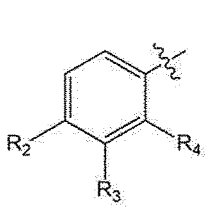 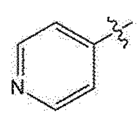 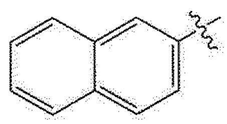

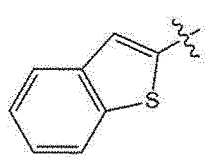 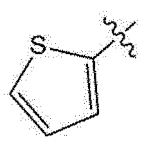 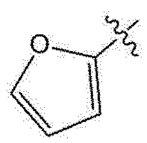

(wherein $R_2$, $R_3$ and $R_4$ are each independently hydrogen, halogen, $C_1$-$C_5$ alkyl, $C_1$-$C_5$ alkoxy, CN or $NO_2$).

5. The compound, a stereoisomer or a pharmaceutically acceptable salt thereof according to the above 1, wherein

the compound is selected from the group consisting of the following compounds:

tert-butyl-4-((4-methylbenzoyl)carbamoyl)piperazine-1-carboxylate;
tert-butyl-4-((4-methoxybenzoyl)carbamoyl)piperazine-1-carboxylate;
tert-butyl-4-((4-chlorobenzoyl)carbamoyl)piperazine-1-carboxylate;
tert-butyl-4-(benzoylcarbamoyl)piperazine-1-carboxylate;
N-(4-methylbenzoyl)piperazine-1-carboxamide;
N-(4-methylbenzoyl)piperazine-1 -carboxamide-2,2,2-trifluoroacetate;
N-(4-methoxybenzoyl)piperazine-1-carboxamide-2,2,2-trifluoroacetate;
N-(4-chlorobenzoyl)piperazine-1-carboxamide-2,2,2-trifluoroacetate;
N-benzoylpiperazine-1-carboxamide-2,2,2-trifluoroacetate;
N-(2-naphthoyl)piperazine-1-carboxamide-2,2,2-trifluoroacetate;
N-(4-cyanobenzoyl)piperazine-1-carboxamide-2,2,2-trifluoroacetate;
N-(furan-2-carbonyl)piperazine-1-carboxamide-2,2,2-trifluoroacetate;
N-(4-nitrobenzoyl)piperazine-1-carboxamide-2,2,2-trifluoroacetate;
N-(2-naphthoyl)piperazine-1-carboxamide hydrochloride;
N-(4-cyanobenzoyl)piperazine-1-carboxamide hydrochloride;
N-(4-nitrobenzoyl)piperazine-1-carboxamide hydrochloride;
N-benzoylpiperazine-1-carboxamide hydrochloride;
N-(furan-2-carbonyl)piperazine-1-carboxamide hydrochloride;
N-(4-methylbenzoyl)piperazine-1-carboxamide hydrochloride;
N-(4-methoxybenzoyl)piperazine-1-carboxamide hydrochloride;
N-(4-chlorobenzoyl)piperazine-1-carboxamide hydrochloride;
N-(2,4-dichlorobenzoyl)piperazine-1-carboxamide-2,2,2-trifluoroacetate;
N-(4-bromobenzoyl)piperazine-1-carboxamide-2,2,2-trifluoroacetate;
N-(thiophene-2-carbonyl)piperazine-1-carbxamide-2,2,2-trifluoroacetate;
N-(benzo[b]thiophene-2-carbonyl)piperazine-1-carboxamide-2,2,2-trifluoroacetate;
N-(4-fluorobenzoyl)piperazine-1-carboxamide-2,2,2-trifluoroacetate;
N-isonicotinoylpiperazine-1-carboxamide-2,2,2-trifluoroacetate;
N-(2,4-dichlorobenzoyl)piperazine-1-carboxamide hydrochloride;
N-(4-bromobenzoyl)piperazine-1-carboxamide hydrochloride;
N-(benzo[b]thiophene-2-carbonyl)piperazine-1-carboxamide hydrochloride;
N-isonicotinoylpiperazine-1-carboxamide hydrochloride;
N-(4-fluorobenzoyl)piperazine-1-carboxamide hydrochloride;
N-(thiophene-2-carbonyl)piperazine-1-carboxamide hydrochloride;
N-(3-chlorobenzoyl)piperazine-1-carboxamide hydrochloride;
N-(3-methylbenzoyl)piperazine-1-carboxamide hydrochloride;
N-(3-methoxybenzoyl)piperazine-1-carboxamide hydrochloride;
N-(3-chlorobenzoyl)piperazine-1-carboxamide-2,2,2-trifluoroacetate;
N-(3-methylbenzoyl)piperazine-1-carboxamide-2,2,2-trifluoroacetate; and
N-(3-methoxybenzoyl)piperazine-1-carboxamide-2,2,2-trifluoroacetate.

6. A pharmaceutical composition for preventing or treating emotional and behavioral disorder-related symptoms, the pharmaceutical composition including the compound, a stereoisomer or a pharmaceutically acceptable salt thereof as defined in any one of the above 1 to 5.
7. The pharmaceutical composition, wherein the above 6, wherein the emotional and behavioral disorder-related symptoms are any one selected from the group consisting of lack of sociability, lack of social cognitive intelligence, stereotyped behavior, hyperactivity, impulsivity and distraction.
8. A pharmaceutical composition for preventing or treating emotional and behavioral disorders, the pharmaceutical composition including the compound, a stereoisomer or a pharmaceutically acceptable salt thereof as defined in any one of the above 1 to 5.
9. A pharmaceutical composition for preventing or treating degenerative neurological diseases, the pharmaceutical composition including the compound, a stereoisomer or a pharmaceutically acceptable salt thereof as defined in any one of the above 1 to 5.
10. The pharmaceutical composition according to the above 8, wherein the emotional and behavioral disorder is any one selected from the group consisting of autism spectrum disorder, schizophrenia, obsessive-compulsive disorder, depression, anxiety disorder, panic disorder and attention deficit hyperactivity disorder.

[Advantageous effects]

The novel compound of the present invention, a stereoisomer or a pharmaceutically acceptable salt thereof acts as an agmatinase inhibitor and is effective in improving emotional and behavioral disorders, as well as symptoms related thereto.

[Brief Description of Drawings]

FIG. 1 shows the agmatinase inhibitory activity of some of the compounds of the present invention shown in Tables 1 to 7.
FIG. 2 shows results confirming the social improvement effects of VPA mice by treatment with the compound of the present invention.
FIG. 3 shows results confirming the cognitive improvement effects of VPA mice by treatment with the compound of the present invention.
FIG. 4 shows a self-grooming test performed using a valproic acid (VPA)-induced an autism animal model.
FIGS. 5 and 6 show neuronal cell protective effects by treatment with the compound of the present invention.

[Mode for Carrying out Invention]

Hereinafter, the present invention will be described in detail.
All technical terms used in the present invention are used in the same meaning as those skilled in the art may generally understand in the related field of the present invention unless otherwise defined. Further, although preferred methods or samples will be described in the present specification, those similar or equivalent are also included in the scope of the present invention.
The present invention relates to a compound represented by Formula 1 below, a stereoisomer or a pharmaceutically acceptable salt thereof:

[Formula 1]

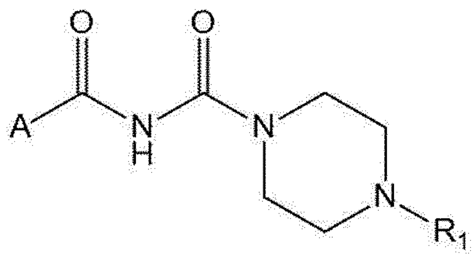

In the above formula, if any substituent is not indicated in a site although the site needs a substituent, it means that a hydrogen substituent is omitted, which would be applied to all formulae in the present invention.
In the above formula, $R_1$ is hydrogen or tert-butyloxycarbonyl group.
In the above formula, A is a single or double cyclic group of $C_5$-$C_{10}$, and may be, for example, benzene, naphthalene, indene, cyclopenta-1,3-diene, cyclohexane, tetrahydronaphthalene, etc., but it is limited thereto.
Each ring of the cyclic group may be substituted with 1 to 3 heteroatoms, and the heteroatoms refer to atoms capable of constituting a ring among atoms other than C. For example, 1 to 3 heteroatoms may be each independently substituted with any one or more selected from the group consisting of N, S and O, they are not limited thereto.
In the cyclic group, sites at which the heteroatom can be substituted may specifically be the same as $Q_1$ to $Q_3$ in the following listed structures, but they are not limited thereto.

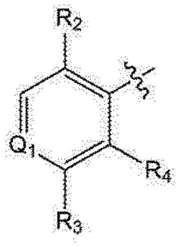 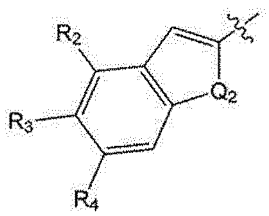 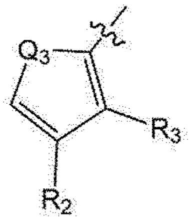

The cyclic group may be substituted with halogen, $C_1$-$C_5$ alkyl, $C_1$-$C_5$ alkoxy, CN or $NO_2$, and may be, for example, F, Cl, Br, methyl group (Me), ethyl group (Et), methoxy group (OMe), ethoxy group (OEt), CN, $NO_2$, and the like, but it is not limited thereto.

In the cyclic group, the sites at which the heteroatom can be substituted with halogen, $C_1$-$C_5$ alkyl, $C_1$-$C_5$ alkoxy, CN or $NO_2$ may be the same as $R_2$ to $R_4$, but they are not limited thereto.

According to one embodiment of the present invention, A may be selected from the following cyclic groups.

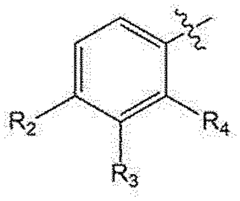 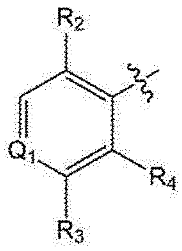 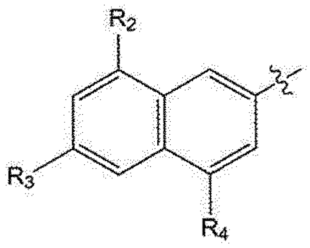

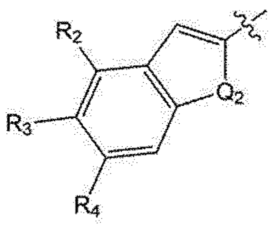 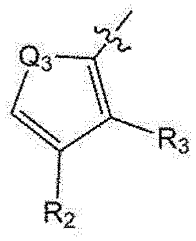

wherein $R_2$, $R_3$ and $R_4$ are each independently hydrogen, halogen, $C_1$-$C_5$ alkyl, $C_1$-$C_5$ alkoxy, CN or $NO_2$, and $Q_1$, $Q_2$ and $R_3$ are each independently N, O or S).

In addition, according to one embodiment of the present invention, more specifically, A may be selected from the following cyclic groups.

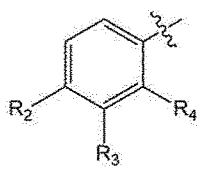 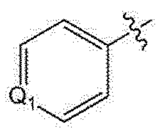 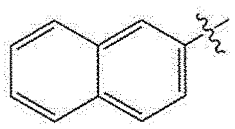

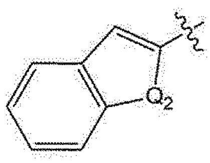 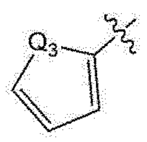

(wherein $R_2$, $R_3$ and $R_4$ are each independently hydrogen, halogen, $C_1$-$C_5$ alkyl, $C_1$-$C_5$ alkoxy, CN or $NO_2$, and $Q_1$, $Q_2$ and $Q_3$ are each independently N, O or S).

Further, according to one embodiment of the present invention, most specifically, A may be selected from the following cyclic groups.

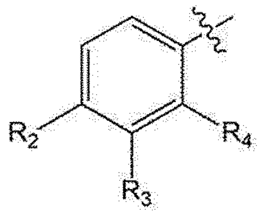
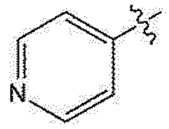
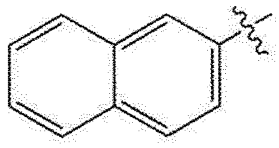
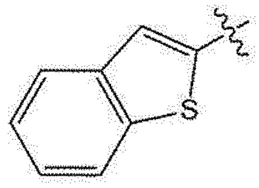
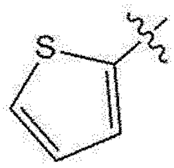
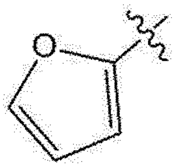

(Wherein $R_2$, $R_3$ and $R_4$ are each independently hydrogen, halogen, $C_1$-$C_5$ alkyl, $C_1$-$C_5$ alkoxy, CN or $NO_2$).

Tables 1 to 7 below show examples of the structure of the compound represented by Formula 1 through a combination of $R_1$ and A in the compound represented by Formula 1 and some of its pharmaceutically acceptable salts. The order of the following tables means the compound number of this specification.

[TABLE 1]

| No. | A | $R_1$ | $R_2$ | $R_3$ | $R_4$ | $Q_1$ | $Q_2$ | $Q_3$ | Salt |
|---|---|---|---|---|---|---|---|---|---|
| 1 | 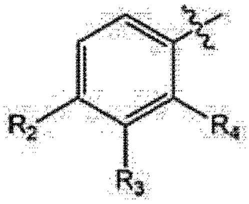 | Boc | Me | H | H | - | - | - | - |
| 2 | | Boc | Et | H | H | - | - | - | - |
| 3 | | Boc | OMe | H | H | - | - | - | - |
| 4 | | Boc | OEt | H | H | - | - | - | - |
| 5 | | Boc | Cl | H | H | - | - | - | - |
| 6 | | Boc | H | H | H | - | - | - | - |
| 7 | | Boc | $NO_2$ | H | H | - | - | - | - |
| 8 | | Boc | CN | H | H | - | - | - | - |
| 9 | | Boc | H | Me | H | - | - | - | - |
| 10 | | Boc | H | Et | H | - | - | - | - |
| 11 | | Boc | H | OMe | H | - | - | - | |
| 12 | | Boc | H | OEt | H | - | - | - | - |
| 13 | | Boc | H | Cl | H | - | - | - | - |
| 14 | | Boc | H | $NO_2$ | H | - | - | - | - |
| 15 | | Boc | H | H | Me | - | - | - | - |
| 16 | | Boc | H | H | OMe | - | - | - | - |
| 17 | | Boc | H | H | C1 | - | - | - | - |
| 18 | | Boc | H | H | $NO_2$ | - | - | - | - |
| 19 | | Boc | Me | Cl | H | - | - | - | - |
| 20 | | Boc | H | Me | Cl | - | - | - | - |
| 21 | | Boc | Me | H | He | - | | - | |
| 22 | | Boc | Cl | Br | H | - | - | - | - |
| 23 | | Boc | $NO_2$ | Cl | H | - | | - | - |
| 24 | | Boc | H | OMe | Br | - | - | - | - |
| 25 | | Boc | Me | OMe | H | - | - | - | - |
| 26 | | Boc | H | OMe | Br | - | - | - | |
| 27 | | Boc | Me | H | Me | - | - | - | - |
| 28 | | H | Me | H | H | - | - | - | - |
| 29 | | H | Me | H | H | - | - | - | $CF_3COOH$ |
| 30 | | H | Me | H | H | - | - | - | HCl |
| 31 | | H | Et | H | H | | | | |
| 32 | | H | OMe | H | H | - | - | - | $CF_3COOH$ |
| 33 | | H | OMe | H | H | - | - | - | HCl |
| 34 | | H | OEt | H | H | - | - | - | - |
| 35 | | H | Cl | H | H | - | - | - | $CF_3COOH$ |

[TABLE 2]

| No. | A | $R_1$ | $R_2$ | $R_3$ | $R_4$ | $Q_1$ | $Q_2$ | $Q_3$ | Salt |
|---|---|---|---|---|---|---|---|---|---|
| 36 | 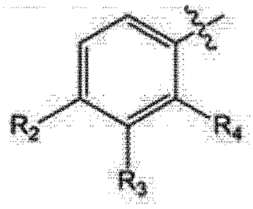 | H | Cl | H | H | - | - | - | HCl |
| 37 | | H | H | H | H | - | - | - | CFgCOOH |
| 38 | | H | H | H | H | - | - | - | HCl |
| 39 | | H | CH | H | H | - | - | - | $CF_3COOH$ |
| 40 | | H | CN | H | H | - | - | - | HCl |
| 41 | | H | $NO_2$ | H | H | - | - | - | $CF_3COOH$ |
| 42 | | H | $NO_2$ | H | H | - | - | - | HCl |
| 43 | | H | Cl | H | Cl | - | - | - | $CF_3COOH$ |
| 44 | | H | Cl | H | Cl | - | - | - | HCl |
| 45 | | H | Br | H | H | - | - | - | $CF_3COOH$ |
| 46 | | H | Br | H | H | - | - | - | HCl |
| 47 | | H | P | H | H | - | - | - | $CF_3COOH$ |
| 48 | | H | F | H | H | - | - | | HCl |
| 49 | | H | H | F | H | - | - | - | - |
| 50 | | H | H | Br | H | - | - | - | - |
| 51 | | H | H | Cl | H | - | - | - | HCl |
| 52 | | H | H | Cl | H | - | - | - | $CF_3COOH$ |
| 53 | | H | H | Me | H | - | - | - | HCl |
| 54 | | H | H | Me | H | - | - | - | $CF_3COOH$ |
| 55 | | H | H | OMe | H | - | - | - | HCl |
| 56 | | H | H | OMe | H | - | - | - | $CF_3COOH$ |
| 57 | | H | Me | Br | H | - | - | - | - |
| 58 | | H | OMe | Cl | H | - | - | - | - |
| 59 | | H | H | Me | OMe | - | - | - | - |
| 60 | | H | Me | Cl | H | - | - | - | - |
| 61 | | H | H | Me | Cl | - | - | - | - |
| 62 | | H | Me | H | Me | - | - | - | - |
| 63 | | H | Cl | Br | H | - | - | - | - |
| 64 | | H | $NO_2$ | Cl | H | - | - | - | - |
| 65 | | H | H | OMe | Br | - | - | - | - |
| 66 | | H | Me | OMe | H | - | - | - | - |
| 67 | | H | H | OMe | Br | - | - | - | - |
| 68 | | H | H | H | Cl | - | - | - | - |
| 69 | | H | H | H | Br | - | - | - | - |
| 70 | | H | H | H | Me | - | - | - | - |
| 71 | | H | H | H | OMe | - | - | - | - |
| 72 | | H | H | H | CN | - | - | - | - |
| 73 | | H | H | OMe | CN | - | - | - | - |
| 74 | | H | H | Me | Br | - | - | - | - |
| 75 | | H | H | Me | Cl | - | - | - | - |

[TABLE 3]

| No. | A | $R_1$ | $R_2$ | $R_3$ | $R_4$ | $Q_1$ | $Q_2$ | $Q_3$ | Salt |
|---|---|---|---|---|---|---|---|---|---|
| 76 | 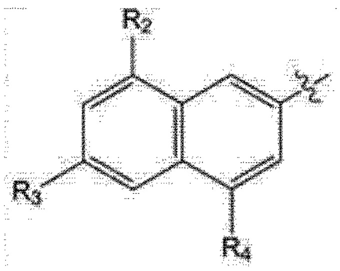 | H | H | H | H | - | - | - | $CF_3COOH$ |
| 77 | | H | H | H | H | - | - | - | HCl |
| 78 | | H | Me | H | H | - | - | - | - |
| 79 | | H | Et | H | H | - | - | - | - |
| 80 | | H | OMe | H | H | - | - | - | - |
| 81 | | H | F | H | H | - | - | - | - |
| 82 | | H | Cl | H | H | - | - | - | - |
| 83 | | H | H | He | H | - | - | - | - |
| 84 | | H | H | OMe | H | - | - | - | - |
| 85 | | H | H | Cl | H | - | - | - | - |
| 86 | | H | H | H | Me | - | - | - | - |
| 67 | | H | H | H | OMe | - | - | - | - |
| as | | H | H | H | Cl | - | - | - | - |
| 89 | | H | Cl | H | Cl | - | - | - | - |
| 90 | | H | Me | Cl | H | - | - | - | - |
| 91 | | H | OMe | Cl | H | - | - | - | - |
| 92 | | Boc | H | H | H | - | - | - | - |
| 93 | | Boc | Me | H | H | - | - | | |
| 94 | | Boc | Et | H | H | - | - | | |
| 95 | | Boc | OMe | H | H | - | - | - | - |
| 96 | | Boc | Cl | H | H | - | - | - | |
| 97 | | Boc | H | Me | H | - | - | - | - |
| 98 | | Boc | H | OMe | H | - | - | - | - |
| 99 | | Boc | H | Cl | H | - | - | - | - |
| 100 | | Boc | H | H | He | - | - | - | - |
| 101 | | Boc | H | H | OMe | - | - | - | - |

[TABLE 4]

| No. | A | $R_1$ | $R_2$ | $R_3$ | $R_4$ | $Q_1$ | $Q_2$ | $Q_3$ | Salt |
|---|---|---|---|---|---|---|---|---|---|
| 102 | 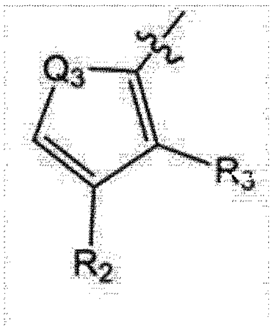 | H | H | H | - | - | - | 0 | $CF_3COOH$ |
| 103 | | H | H | H | - | - | - | 0 | HCl |
| 104 | | H | H | H | - | - | - | S | $CF_4COOH$ |
| 105 | | H | H | H | - | - | | S | HCl |
| 106 | | H | H | H | - | - | - | H | - |
| 107 | | H | Me | H | - | - | - | 0 | - |
| 108 | | H | OMe | H | - | - | - | 0 | - |
| 109 | | H | Cl | H | - | - | - | 0 | - |
| 110 | | H | H | Me | - | - | - | 0 | - |
| 111 | | H | H | OMe | - | - | - | 0 | - |
| 112 | | H | H | CN | - | - | - | 0 | - |
| 113 | | H | Me | H | - | - | - | S | - |
| 114 | | H | OMe | H | - | - | - | S | - |
| 115 | | H | Cl | H | - | - | - | S | - |
| 116 | | H | H | Me | - | - | - | S | - |
| 117 | | H | H | OMe | - | - | - | S | - |
| 118 | | Boc | H | H | - | - | - | 0 | - |
| 119 | | Boc | H | H | - | - | - | S | - |
| 120 | | Boc | H | H | - | - | - | N | - |
| 121 | | Boc | Me | H | - | - | - | 0 | - |
| 122 | | Boc | OMe | H | - | - | - | 0 | |
| 123 | | Boc | Cl | H | - | - | - | 0 | - |
| 124 | | Boc | Me | H | - | - | - | S | - |
| 125 | | Boc | OMe | H | - | - | - | S | - |
| 126 | | Boc | Cl | H | - | - | - | S | - |
| 127 | | Boc | H | Me | - | - | - | 0 | - |
| 128 | | Boc | H | Cl | - | - | - | 0 | - |

[TABLE 5]

| No. | A | $R_1$ | $R_2$ | $R_3$ | $R_4$ | $Q_1$ | $Q_2$ | $Q_3$ | Salt |
|---|---|---|---|---|---|---|---|---|---|
| 129 | 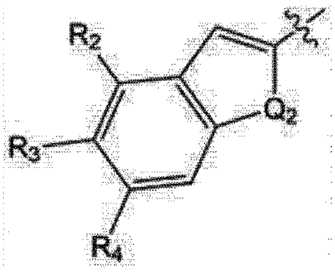 | H | H | H | H | - | S | - | $CF_3COOH$ |
| 130 | | H | H | H | H | - | S | - | HCl |
| 131 | | H | H | H | H | - | 0 | - | - |
| 132 | | H | H | H | H | - | N | - | - |
| 133 | | H | Me | H | H | - | S | - | - |
| 134 | | H | OMe | H | H | - | S | - | - |
| 135 | | H | Cl | H | H | - | S | - | - |
| 136 | | H | H | He | H | - | S | - | - |
| 137 | | H | H | OMe | H | - | S | - | - |
| 138 | | H | H | CN | H | - | S | - | |
| 139 | | H | H | H | Me | - | S | - | - |
| 140 | | H | a | H | OMe | - | S | - | - |
| 141 | | H | H | H | Cl | - | S | - | - - |
| 142 | | Boc | H | H | H | - | S | - | - |
| 143 | | Boc | H | H | H | - | 0 | - | - |
| 144 | | Boc | H | H | H | - | N | - | - |
| 145 | | Boc | Me | H | H | - | S | - | - |
| 146 | | Boc | OMe | H | H | - | S | - | - |
| 1 47 | | Boc | Cl | H | H | - | S | - | - |
| 148 | | Boc | H | Me | H | - | S | - | - |
| 149 | | Boc | H | OMe | H | - | S | - | - |
| 150 | | Boc | H | CN | H | - | S | - | - |
| 151 | | Boc | H | H | Me | - | S | - | - |
| 152 | | Boc | H | H | OMe | - | S | - | - |
| 153 | | Boc | H | H | Cl | - | S | - | - |

[TABLE 6]

| No. | A | $R_1$ | $R_2$ | $R_3$ | $R_4$ | $Q_1$ | $Q_2$ | $Q_3$ | Salt |
|---|---|---|---|---|---|---|---|---|---|
| 154 | 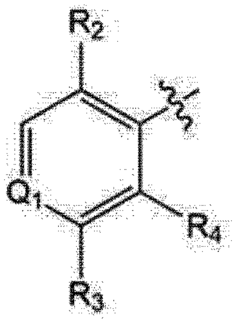 | H | H | H | H | N | - | - | $CF_3COOH$ |
| 155 | | H | H | H | H | N | - | - | HCl |
| 156 | | H | H | H | H | 0 | - | - | - |
| 157 | | H | H | H | H | S | - | - | - |
| 158 | | H | Me | H | H | H | - | - | - |
| 159 | | H | Et | H | H | N | - | - | - |
| 160 | | H | OMe | H | H | N | - | - | - |
| 161 | | H | OEt | H | H | N | - | - | - |
| 162 | | H | Cl | H | H | N | - | - | - |
| 163 | | H | Me | H | H | 0 | - | - | - |
| 164 | | B | Et | H | H | 0 | | | - |
| 165 | | H | OMe | H | H | 0 | - | - | - |
| 166 | | B | OEt | H | H | 0 | - | - | - |
| 167 | | H | Cl | H | H | 0 | - | - | - |
| 168 | | H | Me | H | H | S | - | - | - |
| 169 | | H | Et | H | H | S | - | - | - |
| 170 | | H | OMe | H | H | S | - | - | - |
| 171 | | H | OEt | H | H | S | - | - | - |
| 172 | | H | Cl | H | H | S | - | - | - |
| 173 | | H | H | OMe | H | N | - | - | - |
| 174 | | H | H | OEt | H | N | - | - | - |
| 115 | | H | H | Cl | B | N | - | - | - |
| 176 | | H | H | $NO_2$ | H | N | - | - | - |
| 177 | | H | H | H | He | N | - | - | - |
| 178 | | H | H | H | OMe | N | - | - | - |
| 179 | | H | H | H | Cl | N | - | - | - |
| 180 | | H | H | H | $NO_2$ | N | - | - | - |
| 181 | | H | Me | Cl | H | N | - | - | - |
| 182 | | H | H | Me | Cl | N | - | - | - |
| 183 | | H | Me | H | Me | N | - | - | - |
| 184 | | H | Cl | Br | H | N | - | - | - |

[TABLE 7]

| No. | A | $R_1$ | $R_2$ | $R_3$ | $R_4$ | $Q_1$ | $Q_2$ | $Q_3$ | Salt |
|---|---|---|---|---|---|---|---|---|---|
| 185 | 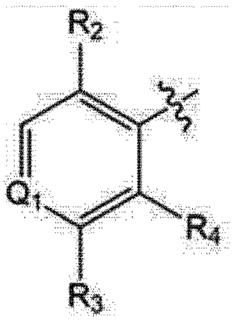 | Boc | H | H | H | N | - | - | - |
| 186 | | Boc | H | H | H | O | - | - | - |
| 187 | | Boc | H | H | H | S | - | - | - |
| 188 | | Boc | Me | H | H | N | - | - | - |
| 189 | | Boc | Et | H | H | N | - | - | - |
| 190 | | Boc | OMe | H | H | N | - | - | - |
| 191 | | Boc | OEt | H | H | N | - | - | - |
| 192 | | Boc | Cl | H | H | N | - | - | - |
| 193 | | Boc | Me | H | H | O | - | - | - |
| 194 | | Boc | Et | H | H | O | - | - | - |
| 195 | | Boc | OMe | H | H | O | - | - | - |
| 196 | | Boc | OEt | H | H | O | - | - | - |
| 197 | | Boc | Cl | H | H | O | - | - | - |
| 198 | | Boc | He | H | H | S | - | - | - |
| 199 | | Boc | Et | H | H | S | - | - | - |
| 200 | | Boc | OMe | H | H | S | - | - | - |
| 201 | | Boc | OEt | H | H | S | - | - | - |
| 202 | | Boc | Cl | H | H | S | - | - | - |
| 203 | | Boc | H | OMe | H | N | - | - | - |
| 204 | | Boc | H | OEt | H | N | - | - | - |
| 205 | | Boc | H | Cl | H | N | - | - | - |
| 206 | | Boc | H | $NO_2$ | H | N | - | - | - |
| 207 | | Boc | H | H | Me | N | - | - | - |
| 208 | | Boc | H | H | 0Me | N | - | - | - |
| 209 | | Boc | H | H | Cl | N | - | - | - |
| 210 | | Boc | H | H | $NO_2$ | N | - | - | - |
| 211 | | Boc | Me | Cl | H | H | - | - | - |
| 212 | | Boc | H | Me | Cl | N | - | - | - |
| 213 | | Boc | He | H | Me | N | - | - | - |
| 214 | | Boc | Cl | Br | H | N | - | - | - |

The present invention relates to a compound selected from the group consisting of the following compounds, and a stereoisomer or a pharmaceutically acceptable salt thereof:

tert-butyl-4-((4-methylbenzoyl)carbamoyl)piperazine-1-carboxylate;
tert-butyl-4-((4-methoxybenzoyl)carbamoyl)piperazine-1-carboxylate;
tert-butyl-4-((4-chlorobenzoyl)carbamoyl)piperazine-1-carboxylate;
tert-butyl-4-(benzoylcarbamoyl)piperazine-1-carboxylate;
N-(4-methylbenzoyl)piperazine-1-carboxamide;
N-(4-methylbenzoyl)piperazine-1-carboxamide-2,2,2-trifluoroacetate;
N-(4-methoxybenzoyl)piperazine-1-carboxamide-2,2,2-trifluoroacetate;

N-(4-chlorobenzoyl)piperazine-1-carboxamide-2,2,2-trifluoroacetate;
N-benzoylpiperazine-1-carboxamide-2,2,2-trifluoroacetate;
N-(2-naphthoyl)piperazine-1-carboxamide-2,2,2-trifluoroacetate;
N-(4-cyanobenzoyl)piperazine-1-carboxamide-2,2,2-trifluoroacetate;
N-(furan-2-carbonyl)piperazine-1-carboxamide-2,2,2-trifluoroacetate;
N-(4-nitrobenzoyl)piperazine-1-carboxamide-2,2,2-trifluoroacetate;
N-(2-naphthoyl)piperazine-1-carboxamide hydrochloride;
N-(4-cyanobenzoyl)piperazine-1-carboxamide hydrochloride;
N-(4-nitrobenzoyl)piperazine-1-carboxamide hydrochloride;
N-benzoylpiperazine-1-carboxamide hydrochloride;
N-(furan-2-carbonyl)piperazine-1-carboxamide hydrochloride;
N-(4-methylbenzoyl)piperazine-1-carboxamide hydrochloride;
N-(4-methoxybenzoyl)piperazine-1-carboxamide hydrochloride;
N-(4-chlorobenzoyl)piperazine-1-carboxamide hydrochloride;
N-(2,4-dichlorobenzoyl)piperazine-1-carboxamide-2,2,2-trifluoroacetate;
N-(4-bromobenzoyl)piperazine-1-carboxamide-2,2,2-trifluoroacetate;
N-(thiophene-2-carbonyl)piperazine-1-carbxamide-2,2,2-trifluoroacetate;
N-(benzo[b]thiophene-2-carbonyl)piperazine-1-carboxamide-2,2,2-trifluoroacetate;
N-(4-fluorobenzoyl)piperazine-1-carboxamide-2,2,2-trifluoroacetate;
N-isonicotinoylpiperazine-1-carboxamide-2,2,2-trifluoroacetate;
N-(2,4-dichlorobenzoyl)piperazine-1-carboxamide hydrochloride;
N-(4-bromobenzoyl)piperazine-1-carboxamide hydrochloride;
N-(benzo[b]thiophene-2-carbonyl)piperazine-1-carboxamide hydrochloride;
N-isonicotinoylpiperazine-1-carboxamide hydrochloride;
N-(4-fluorobenzoyl)piperazine-1-carboxamide hydrochloride;
N-(thiophene-2-carbonyl)piperazine-1-carboxamide hydrochloride;
N-(3-chlorobenzoyl)piperazine-1-carboxamide hydrochloride;
N-(3-methylbenzoyl)piperazine-1-carboxamide hydrochloride;
N-(3-methoxybenzoyl)piperazine-1-carboxamide hydrochloride;
N-(3-chlorobenzoyl)piperazine-1-carboxamide-2,2,2-trifluoroacetate;
N-(3-methylbenzoyl)piperazine-1-carboxamide-2,2,2-trifluoroacetate; and
N-(3-methoxybenzoyl)piperazine-1-carboxamide-2,2,2-trifluoroacetate.

Further, the present invention relates to a pharmaceutical composition which includes the compound, a stereoisomer or a pharmaceutically acceptable salt thereof.

Agmatinase is an enzyme that decomposes agmatine, and it was confirmed that a content of the agmatine is reduced in some patients with autism, which is determined to be due to excessive action of the agmatinase. Further, this condition is predicted to suppress excessive excitatory nerve activity, and thus, it is expected to have preventive or therapeutic effects on emotional and behavioral disorders, as well as symptoms related thereto. Since the compound of the present invention has an agmatinase inhibitory activity, it could be understood that the above effects are expected.

The pharmaceutical composition may be a pharmaceutical composition for preventing or treating emotional and behavioral disorder-related symptoms, and specifically, the emotional and behavioral disorder-related symptoms may include lack of sociability, lack of social cognitive intelligence, stereotyped behavior, hyperactivity, impulsivity and distraction, but it is not limited thereto.

In the present invention, the lack of sociability refers to a condition in which a subject patient cannot form a smooth relationship with people normally in relationships with others who have to contact with the subject patient or are around him or her, or does not have a normal social life. For example, this disease refers to a state where a person does not fit in a relationship with people but is immersed in his or her own world.

In the present invention, the lack of social cognitive intelligence refers to a state that social cognitive intelligence, which is an inherent ability to determine lacking of social behavior helpful to adapt to interpersonal relationships or social life. For example, this refers to a state in which the behavior of understanding and inferring facial expressions, behaviors, emotions, etc. is not exhibited, and the conventions, morality and appropriate behavior in mutual relationships required by the group are not normally performed.

In the present invention, the stereotyped behavior refers to a continuous and repetitive behavior such as shaking the body back and forth, continuously moving the hands, repeating a meaningless sound, etc., a state in which the same behavior is continuously repeated regardless of the surrounding situations.

In the present invention, the hyperactivity is also referred to as excessive behavior, and refers to abnormally excessively active or moving behavior, etc., and means a state that interferes with learning and causes serious problems

in behavior control.

In the present invention, the impulsivity means a tendency to act suddenly in response to internal impulses without thinking and hardly considering the consequences of actions.

In the present invention, the distraction refers to a state in which one action cannot continuously proceeded, no persistence exists, and a person quickly moves to another activity or is confused about what to do.

Further, the pharmaceutical composition may be a pharmaceutical composition for preventing or treating emotional and behavioral disorders, specifically a pharmaceutical composition for preventing or treating autism spectrum disorder, schizophrenia, obsessive-compulsive disorder, depression, anxiety disorder, panic disorder and attention deficit hyperactivity disorder, but it is not limited thereto.

In the present invention, the autism type disorder is also referred to as autism spectrum disorder, which means a typical developmental disorder that exhibits serious maladaptive disorders. Fundamentally, this show deficiencies in social interaction and communication skills, limited, repetitive, and stereotyped behavioral characteristics, and very diverse characteristics in behaviors, skills, preferences, functioning, and learning that are required according to growth.

In the present invention, the schizophrenia is a disease called schizophrenia in the past, and refers to a mental disease that causes a wide range of clinical abnormalities across various aspects of personality, such as thinking, emotion, perception, behavior and the like. The schizophrenia appears in different types, and it is not a single disease but is identified as a disease group consisting of several diseases with common characteristics.

In the present invention, the obsessive-compulsive disorder refers to a subtype of anxiety disorder in which unwanted thoughts and actions are repeated, in order to reduce obsession and anxiety, which are painful thoughts, impulses, or images that repeatedly penetrate consciousness. Repetitive compulsions are the main symptom. Obsessive compulsive behaviors appear in the form of cleaning behaviors, checking behaviors, repetitive behaviors, tidying behaviors, and procrastinating behaviors. Even though they know that the behavior is undesirable and is beyond the bounds, the behavior is repeated in order to recover from anxiety due to obsessive thinking.

In the present invention, the depression does not mean a state in which only mood is temporarily lowered, but a state in which overall mental functions such as content of thought, thought process, motivation, drive, interest, behavior, sleep, and physical activity are lowered, wherein this state appears almost all day and every day.

In the present invention, the anxiety disorder refers to a mental disorder that causes impairment in daily life due to various forms of abnormal or pathological anxiety and fear, including panic disorder, agoraphobia, social anxiety disorder, specific phobia, separation anxiety disorder and the like.

In the present invention, the panic disorder is a kind of anxiety disorder, and refers to an anxiety disorder in which panic attacks, including intense anxiety that suddenly surges and intense fear that will soon die, appear repeatedly.

In the present invention, the attention deficit hyperactivity disorder, also commonly referred to as ADHD, is a mental disease in which distraction, hyperactivity, and impulsivity are seen as main symptoms, and is characterized by onset in early childhood and following a chronic course

Further, the pharmaceutical composition may be a pharmaceutical composition for preventing or treating degenerative neurological diseases.

Since the compound of the present invention can improve symptoms of sociability and social cognitive ability and has an effect of protecting nerve cells from cell damage and death caused by oxidative stress, it can exhibit medicinal effects against various degenerative neurological diseases.

The neurodegenerative diseases may include, for example, Alzheimer's disease, Parkinson's disease, multiple sclerosis, neuroblastoma, stroke, Lou Gehrig's disease, Huntington's disease, Pick's disease, Creutzfeldt-Jakob disease, post-traumatic stress disorder, depression, schizophrenia, autoimmune diseases of the nervous system, frontal temporal dementia, Lewy dementia, cortical hypoplasia, amyotrophic lateral sclerosis, and memory and cognitive decline, but they are not limited thereto.

In the present invention, the pharmaceutical composition may be prepared using a pharmaceutically suitable and physiologically acceptable additive in addition to the active ingredient, which is the compound of the present invention. The composition may be administered to a mammal. As the additive described above, for example, excipients, disintegrants, sweeteners, binders, coating agents, swelling agents, lubricants, glidants or flavoring agents may be used.

Further, the pharmaceutical composition of the present invention may be preferably formulated as a pharmaceutical composition that includes at least one pharmaceutically acceptable carrier in addition to the active ingredient in a pharmaceutically effective amount described above for administration.

The "pharmaceutically effective amount" means an amount sufficient to treat a disease with a reasonable benefit/risk ratio applicable to medical treatment, and an effective dose level may be determined based on a type and severity of patient's disease, drug activity, drug sensitivity, time of administration, route of administration and rate of excretion, duration of treatment, factors including drugs used concurrently, and other factors well known in the medical field. The pharmaceutical composition according to the present invention may be administered as an individual therapeutic agent or administered in combination with other therapeutic agents. Further, the composition may be administered sequentially or simultaneously with a conventional therapeutic agent, and may be administered in single or multiple

doses. In consideration of all of the above factors, it is important to administer a minimum amount capable of attaining the maximum effect without side effects, such an amount could be easily determined by those skilled in the art.

Specifically, the effective amount of the pharmaceutical composition according to the present invention may vary depending on an age, gender, condition and/or body weight of the patient, absorption of the active ingredient in the body, inactivation rate and excretion rate, type of disease, and the drug to be used in combination. Typically, 0.001 to 150 mg, preferably 0.01 to 100 mg per 1 kg of body weight may be administered daily or every other day, or may be divided into 1 to 3 times a day. However, the dosage may be increased or decreased depending on the route of administration, severity of obesity, gender, body weight, age, etc., therefore, would not limit the scope of the present invention in any way.

Further, the "pharmaceutically acceptable" refers to a composition that is physiologically acceptable and does not usually cause allergic reactions such as gastrointestinal disorders and dizziness, or similar reactions when administered to humans.

Examples of the carrier, excipient and diluent may include lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinylpyrrolidone, water, methyl hydroxybenzoate, propyl hydroxybenzoate, talc, magnesium stearate, and mineral oils. Further, fillers, anti-aggregating agents, lubricants, wetting agents, flavoring agents, emulsifying agents, and preservatives may additionally be included.

Further, the composition of the present invention may be formulated using any method known in the art in order to provide rapid, sustained or delayed release of the active ingredient after administration thereof to a subject in need of treatment using the pharmaceutical composition of the present invention including humans. The formulation may be powder, granule, tablet, emulsion, syrup, aerosol, soft or hard gelatin capsule, sterile injectable solution, sterile powder.

The compound represented by Formula 1 according to the present invention may be prepared by any method known in various documents. In the following preparative examples, the synthetic methods for some of the compounds listed in Table 1 have been briefly described, however, they are not limited thereto.

Hereinafter, the present invention will be described in detail by means of preparative examples and examples of the present invention.

**Preparative Example**

**1. Synthesis of Compound 1**

[Scheme 1]

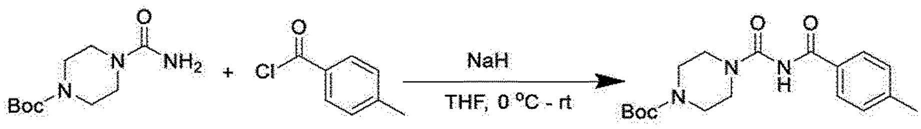


SMI SMII

A flame-dried round bottom flask equipped with a magnetic stirrer was charged with a solution of SMI (1 mmol) and anhydrous THF (10 ml). NaH (5 mmol) was added to RM at 0 °C and stirred for about 2 h under nitrogen conditions. Then, aryl chloride (SMII, 2 mmol) in THF (5 ml) was slowly added to the reaction mixture and stirred overnight. Saturated ammonium chloride solution (7-10 ml) was added to the reaction and extracted with $CH_2Cl_2$. The organic layer was dried over $MgSO_4$, filtered and concentrated in vacuum to yield a white solid. The residue was purified using n-hexane:ethyl acetate (4:1 and 2:1) through flask column chromatography to obtain the desired product as a white solid.

Retention factor: 0.41 (H:E = 1:1)
Yield: 229 mg (66%)
NMR data: 1H NMR (500 MHz, CDCl3): δ 8.44 (s, 1H), 7.76 (d, J = 8.15 Hz, 2H), 7.27-7.26 (m, 2H, overlapped with CDCl3), 3.52 (s, 8H), 2.41 (s, 3H), 1.47 (s, 9H).

**2. Synthesis of Compound 3**

[Scheme 2]

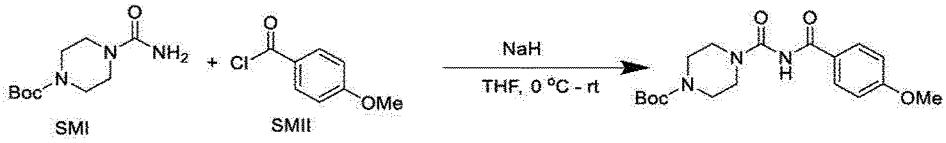


A flame dried round bottom flask equipped with a magnetic stirrer was charged with a solution of SMI (1 mmol) and anhydrous THF (10 ml). NaH (5 mmol) was added to RM at 0 °C and stirred for about 2 h under nitrogen conditions. Then, aryl chloride (SMII, 2 mmol) in THF (5 ml) was slowly added to the reaction mixture and stirred overnight. Saturated ammonium chloride solution (7-10 ml) was added to the reaction and extracted with $CH_2Cl_2$. The organic layer was dried over $MgSO_4$, filtered and concentrated in vacuum to yield a white solid. The residue was purified using n-hexane:ethyl acetate (4:1 and 2:1) through flask column chromatography to obtain the desired product as a white solid.

Retention factor: 0.30 (H:E = 1:1)
Yield: 225 mg (62%)
NMR data: 1H NMR (500 MHz, CDCl3): δ 8.45 (s, 1H), 7.84 (d, *J* = 8.75 Hz, 2H), 6.95 (d, *J* = 8.8 Hz, 2H), 3.87, (s, 3H), 3.52 (s, 8H), 1.47 (s, 9H).

**3. Synthesis of Compound 5**

[Scheme 3]

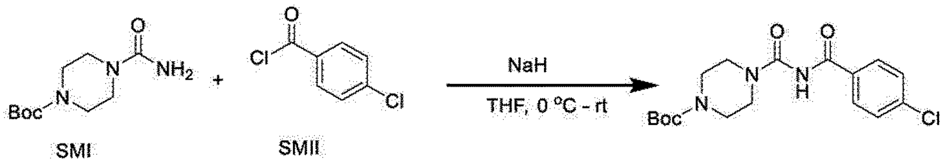


A flame dried round bottom flask equipped with a magnetic stirrer was charged with a solution of SMI (1 mmol) and anhydrous THF (10 ml). NaH (5 mmol) was added to RM at 0 °C and stirred for about 2 h under nitrogen conditions. Then, aryl chloride (SMII, 2 mmol) in THF (5 ml) was slowly added to the reaction mixture and stirred overnight. Saturated ammonium chloride solution (7-10 ml) was added to the reaction and extracted with $CH_2Cl_2$. The organic layer was dried over $MgSO_4$, filtered and concentrated in vacuum to yield a white solid. The residue was purified using n-hexane:ethyl acetate (4:1 and 2:1) through column chromatography to obtain the desired product as a white solid.

Retention factor: 0.43 (H:E = 1:1)
Yield: 220 mg (60%)
NMR data: 1H NMR (500 MHz CDCl3): δ 9.13 (s, 1H), 7.85 (d, *J* = 8.35 Hz, 2H), 7.50 (d, *J* = 8.65 Hz, 2H), 3.52 (s, 8H), 1.48 (s, 9H).

**4. Synthesis of Compound 6**

[Scheme 4]

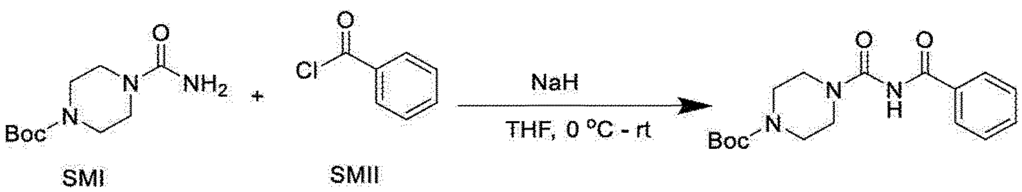

A flame dried round bottom flask equipped with a magnetic stirrer was charged with a solution of SMI (1 mmol) and anhydrous THF (10 ml). NaH (5 mmol) was added to RM at 0 °C and stirred for about 2 h under nitrogen conditions. Then, aryl chloride (SMII, 2 mmol) in THF (5 ml) was slowly added to the reaction mixture and stirred overnight. Saturated ammonium chloride solution (7-10 ml) was added to the reaction and extracted with $CH_2Cl_2$. The organic layer was dried over $MgS_4$, filtered, and concentrated in vacuum to yield a white solid. The residue was purified using n-hexane:ethyl acetate (4:1 and 2:1) through flask chromatography to obtain the desired product as a white solid.

Retention factor: 0.40 (H:E = 1:1)
Yield: 234 (70%)
NMR data: 1H NMR (500 MHz, CDCl3): δ 8.32 (s, 1H), 7.85 (d, *J* = 7.1 Hz, 2H), 7.59-7.56 (m, 1H), 7.49-7.46 (m, 2H), 3.52 (s, 8H), 1.47 (s, 9H).

**5. Synthesis of Compound 28**

[Scheme 5]

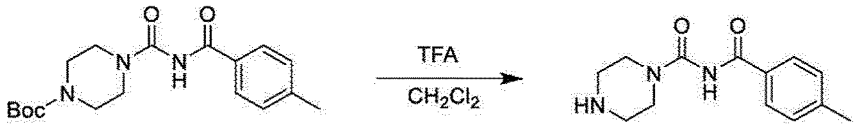


A flame dried round bottom flask equipped with a magnetic stirrer was charged with a solution of anhydrous $CH_2Cl_2$ (3 ml) and SMI (0.28 mmol). TFA (5.6 mmol) was added to RM and stirred overnight. Then, the RM was concentrated in vacuum and dissolved in water. The pH of the RM was made neutral using NaOH and extracted with $CH_2Cl_2$. The RM was dried over $MgSO_4$ and evaporated. Column chromatography was performed using a $CH_2Cl_2$:MeOH concentration gradient to obtain the desired product in a viscous liquid state.

Retention factor: 0.41 (H:E: EtOH = 1:1)
Yield: 14 mg (> 20%)
NMR data: 1H NMR (500 MHz, DMSO): δ 7.75- 7.74 (m, 2H), 7.29-7.27 (m, 4H overlapped with CDCl3), 3.55 (s, 4H), 2.96-2.94 (m, 4H), 2.43 (s, 3H).

**6. Synthesis of Compound 29**

[Scheme 6]

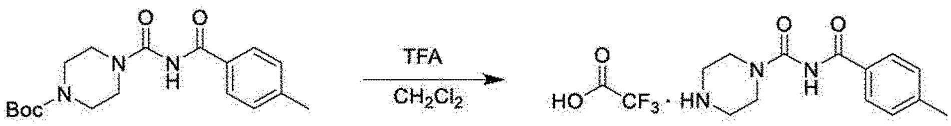


A flame dried round bottom flask equipped with a magnetic stirrer was charged with a solution of anhydrous $CH_2Cl_2$ (3 ml) and SMI (0.32 mmol). TFA (6.4 mmol) was added to RM and stirred overnight. Then, the RM was concentrated in vacuum using methanol and dichloromethane (DCM) to yield a solid product. The obtained product was washed with DCM and filtered to obtain the desired product as a white solid.

Retention factor: NA
Yield: 106 mg (92%)
NMR data: 1H NMR (500 MHz, DMSO): δ 10.31 (s, 1H), 9.24 (s, 2H), 7.79 (d, *J* = 7.7 Hz, 2H), 7.30 (d, *J* = 7.75 Hz, 2H), 3.64 (s, 4H), 3.16 (s, 4H), 2.36 (s, 3H).

**7. Synthesis of Compound 32**

[Scheme 7]

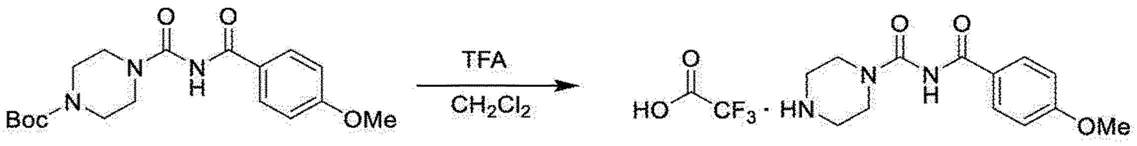


A flame dried round bottom flask equipped with a magnetic stirrer was charged with a solution of anhydrous $CH_2Cl_2$ (3 ml) and SMI (0.27 mmol). TFA (5.4 mmol) was added to RM and stirred overnight. Then, the RM was concentrated in vacuum using methanol and dichloromethane (DCM) to yield a solid product. The obtained product was washed with DCM and filtered to obtain the desired product as a white solid.

Retention factor: NA
Yield: 88 mg (87%)
NMR data: 1H NMR (500 MHz, DMSO): δ 10.26 (s, 1H), 9.27 (s, 2H), 7.88 (d, *J* = 8.45 Hz, 2H), 7.01 (d, *J* = 8.45 Hz, 2H), 3.81 (s, 3H), 3.64 (s, 4H), 3.17 (s, 4H).

**8. Synthesis of Compound 35**

[Scheme 8]

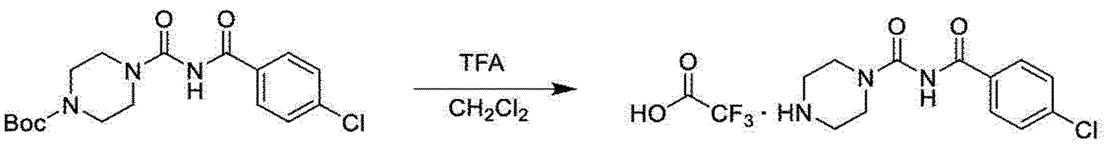


A flame dried round bottom flask equipped with a magnetic stirrer was charged with a solution of anhydrous $CH_2Cl_2$ (3 ml) and SMI (0.32 mmol). TFA (6.4 mmol) was added to RM and stirred overnight. Then, the RM was concentrated in vacuum using methanol and dichloromethane (DCM) to yield a solid product. The obtained product was washed with DCM and filtered to obtain the desired product as a white solid.

Retention factor: NA
Yield: 117 % (96%)
NMR data: 1H NMR (500 MHz, DMSO): δ 10.48 (s, 1H), 9.16 (s, 2H), 7.88 (d, *J* = 8.1 Hz, 2H), 7.57 (d, *J* = 8.1Hz, m, 2H), 3.65-3.63 (m, 4H), 3.16- 3.15 (m, 4H).

**9. Synthesis of Compound 37**

[Scheme 9]

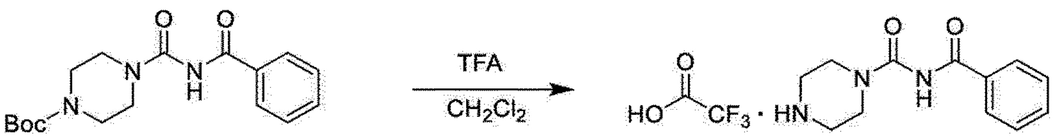

A flame dried round bottom flask equipped with a magnetic stirrer was charged with a solution of anhydrous $CH_2Cl_2$ (5 ml) and SMI (0.42 mmol). TFA (8.4 mmol) was added to RM and stirred overnight. Then, the RM was concentrated in vacuum using methanol and dichloromethane (DCM) to yield a solid product. The obtained product was washed with DCM and filtered to obtain the desired product as a white solid.

Retention factor: NA
Yield: 144 mg (99%)
NMR data: 1H NMR (500 MHz, DMSO): δ 10.38 (s, 1H), 9.01 (s, 2H), 7.87 (d, *J* = 7.4 Hz, 2H), 7.62-7.59 (m, 1H), 7.52-7.49 (m, 2H), 3.65-3.63 (m, 4H), 3.17- 3.15(m, 4H).

**10. Synthesis of Compound 76**

[Scheme 10]

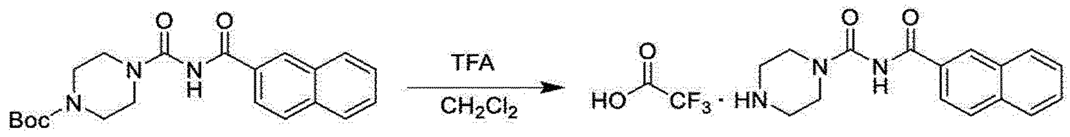


A flame dried round bottom flask equipped with a magnetic stirrer was charged with a solution of anhydrous $CH_2Cl_2$ (3 ml) and SMI (0.31 mmol). TFA (6.2 mmol) was added to RM and stirred overnight. Then, the RM was concentrated in vacuum using methanol and dichloromethane (DCM) to yield a solid product. The obtained product was washed with DCM and filtered to obtain the desired product as a white solid.

Retention factor: NA

Yield: 110 mg (90%)

NMR data: 1H NMR (500 MHz, DMSO): δ 10.53 (s, 1H), 8.89 (s, 2H), 8.50 (s, 1H), 8.00-7.99 (m, 3H), 7.90 (d, *J* = 8.55 Hz, 1 H), 7.66-7.60 (m, 2H), 3.67-3.65 (m, 4H), 3.17 (s, 4H).

**11. Synthesis of Compound 39**

[Scheme 11]

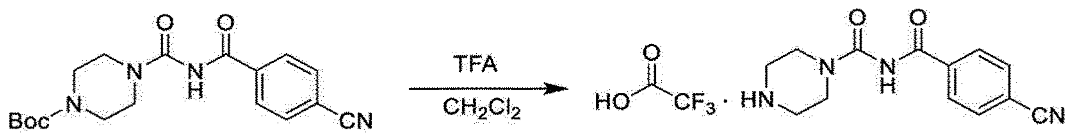


A flame dried round bottom flask equipped with a magnetic stirrer was charged with a solution of anhydrous $CH_2Cl_2$ (3 ml) and SMI (0.39 mmol). TFA (7.8 mmol) was added to RM and stirred overnight. Then, the RM was concentrated in vacuum using methanol and dichloromethane (DCM) to obtain a solid product. The obtained product was washed with DCM and filtered to obtain the desired product as a white solid.

Retention factor: NA
Yield: 132 mg (91%)
NMR data: 1H NMR (500 MHz, DMSO): δ 10.66 (s, 1H), 9.03 (s, 2H), 7.97 (s, 4H), 3.64-3.62 (m, 4H), 3.14 (s, 4H).

**12. Synthesis of Compound 102**

[Scheme 12]

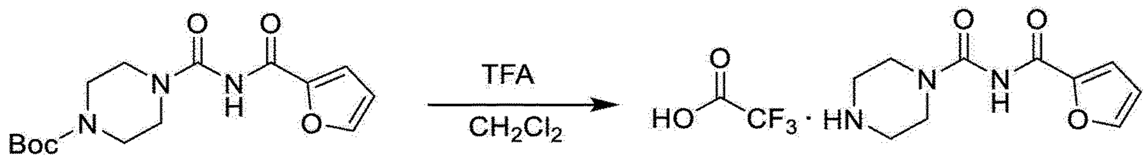


A flame dried round bottom flask equipped with a magnetic stirrer was charged with a solution of anhydrous $CH_2Cl_2$ (5 ml) and SMI (0.43 mmol). TFA (8.6 mmol) was added to RM and stirred overnight. Then, the RM was concentrated in vacuum using methanol and dichloromethane (DCM) to obtain a solid product. The obtained product was washed with DCM and filtered to obtain the desired product as a white solid.

Retention factor: NA
Yield: 123 mg (85%)
NMR data: 1H NMR (500 MHz, DMSO): δ 10.29 (s, 1H), 8.90 (s, 2H), 7.94 (d, *J* = 1.65 Hz, 1H) 7.37 (d, *J* = 3.5 Hz, 1H), 6.69-6.68 (m, 1H), 3.61-3.59 (m, 4H), 3.13 (s, 4H).

**13. Synthesis of Compound 41**

[Scheme 13]

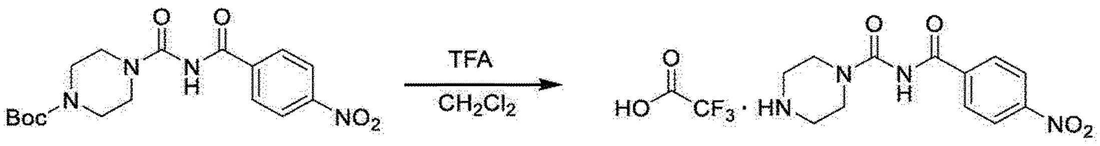


A flame dried round bottom flask equipped with a magnetic stirrer was charged with a solution of anhydrous $CH_2Cl_2$ (3 ml) and SMI (0.37 mmol). TFA (7.4 mmol) was added to RM and stirred overnight. Then, the RM was concentrated in vacuum using methanol and dichloromethane (DCM) to obtain a solid product. The obtained product was washed with DCM and filtered to obtain the desired product as a white solid.

Retention factor: NA
Yield: 135 mg (93%)
NMR data: 1H NMR (500 MHz, DMSO): δ 10.73 (s, 1H), 8.92 (s, 2H), 8.32 (d, *J* = 8.55 Hz, 2H) 8.04 (d, *J* = 8.5 Hz, 2H), 3.65-3.63 (m, 4H), 3.15 (s, 4H).

**14. Synthesis of Compound 77**

[Scheme 14]

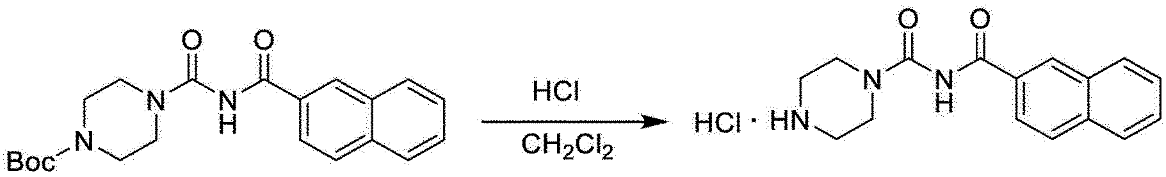

A flame dried round bottom flask equipped with a magnetic stirrer was charged with a solution of anhydrous $CH_2Cl_2$ (2 ml) and SMI (0.31 mmol). HCl (2 mmol) in diethyl ether (3.1 mmol) was added to RM at 0 °C and stirred overnight. Then, the RM was filtered to obtain the desired product as a white solid.

Retention factor: NA
Yield: 89 mg (90%)
NMR data: 1H NMR (500 MHz, DMSO): δ 10.56 (s, 1H), 9.47 (s, 2H), 8.54-8.53 (m, 1H), 8.05- 7.99 (m, 3H), 7.93-7.91 (m, 1H), 7.67-7.60 (m, 2H), 3.72-3.70 (m, 4H), 3.14 (s, 4H); 13C NMR (125 MHz, DMSO): δ 152.6, 134.60, 131.9, 130.41, 129.07, 129.03, 128.2, 128.0, 127.6, 126.9, 124.4, 42.6.

**15. Synthesis of Compound 40**

[Scheme 15]

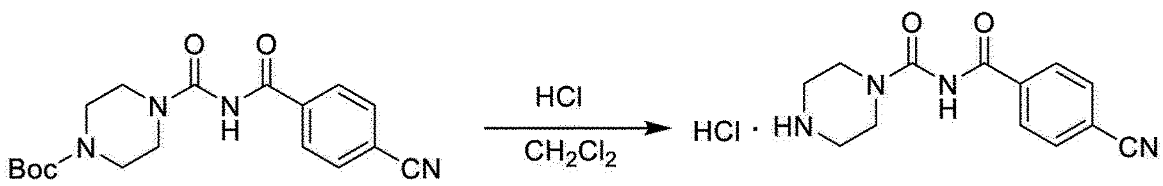


A flame dried round bottom flask equipped with a magnetic stirrer was charged with a solution of anhydrous $CH_2Cl_2$ (2 ml) and SMI (0.33 mmol). HCl (2 mmol) in diethyl ether (3.3 mmol) was added to RM at 0 °C and stirred overnight. Then, the RM was filtered to obtain the desired product as a white solid.

Retention factor: NA
Yield: 88.5 mg (91%)
NMR data: 1H NMR (500 MHz, DMSO): δ 10.70 (s, 1H), 9.44 (s, 2H), 8.00 (s, 4H), 3.69-3.67 (m, 4H), 3.12 (s, 4H); 13C NMR (125 MHz, DMSO): δ 166.1, 152.6, 137.8, 132.8, 129.3, 118.6, 114.8, 43.0.

**16. Synthesis of Compound 42**

[Scheme 16]

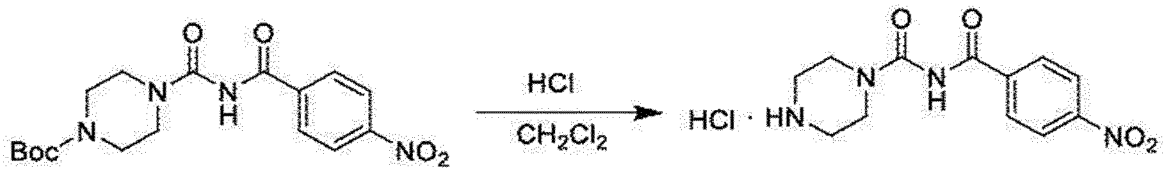


A flame dried round bottom flask equipped with a magnetic stirrer was charged with a solution of anhydrous $CH_2Cl_2$ (5 ml) and SMI (0.37 mmol). HCl (2 mmol) in diethyl ether (3.7 mmol) was added to RM at 0 °C and stirred overnight. Then, the RM was filtered to obtain the desired product as a white solid.

Retention factor: NA
Yield: 107 mg (92%)
NMR data: 1H NMR (500 MHz, DMSO): δ 10.79 (s, 1H), 9.48 (s, 2H), 8.32 (d, *J* = 8.8 Hz, 2H), 8.07 (d, *J* = 8.75 Hz, 2H), 3.71-3.69 (m, 4H), 3.13 (s, 4H); 13C NMR (125 MHz, DMSO): δ166.0, 152.6, 149.9, 139.5, 130.0, 123.9, 43.0.

**17. Synthesis of Compound 38**

[Scheme 17]

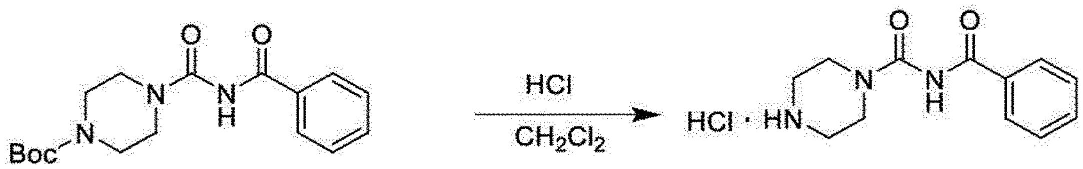


A flame dried round bottom flask equipped with a magnetic stirrer was charged with a solution of anhydrous $CH_2Cl_2$ (2 ml) and SMI (0.33 mmol). HCl (2 mmol) in diethyl ether (3.3 mmol) was added to RM at 0 °C and stirred overnight. Then, the RM was filtered to obtain the desired product as a white solid.

Retention factor: NA
Yield: 83 mg (93.24 %)
NMR data: 1H NMR (500 MHz, DMSO): δ 10.42 (s, 1H), 9.57 (s, 2H), 7.88-7.87 (m, 2H), 7.61- 7.58 (m, 1H), 7.51-7.48 (m, 2H), 3.69-3.67 (m, 4H), 3.13-3.11 (m, 4H).

**18. Synthesis of Compound 103**

[Scheme 18]

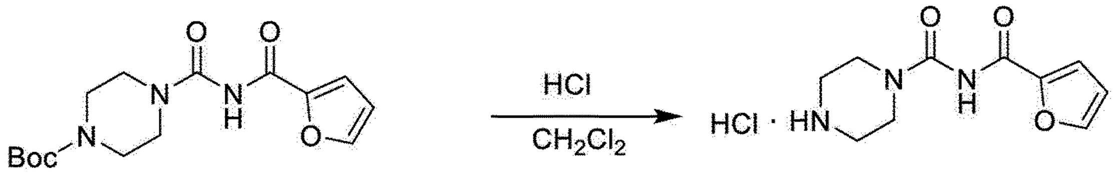


A flame dried round bottom flask equipped with a magnetic stirrer was charged with a solution of anhydrous $CH_2Cl_2$ (2 ml) and SMI (0.40 mmol). HCl (2 mmol) in diethyl ether (4 mmol) was added to RM at 0 °C and stirred overnight. Then, the RM was filtered to obtain the desired product as a white solid.

Retention factor: NA
Yield: 91.5 mg (88%)
NMR data: 1H NMR (500 MHz, DMSO): δ10.32 (s, 1H), 9.49 (s, 2H), 7.93 (d, *J* = 1.5 Hz, 1H), 7.42 (d, *J* = 3.4 Hz, 1H), 6.67 (dd, *J* = 3.55, 1.7 Hz, 1H), 3.66-3.64 (m, 4H), 3.10-3.09 (m, 4H) ; 13C NMR (125 MHz, DMSO): δ 156.6, 151.9, 146.7, 146.1, 116.5, 112.2, 42.5.

**19. Synthesis of Compound 30**

[Scheme 19]

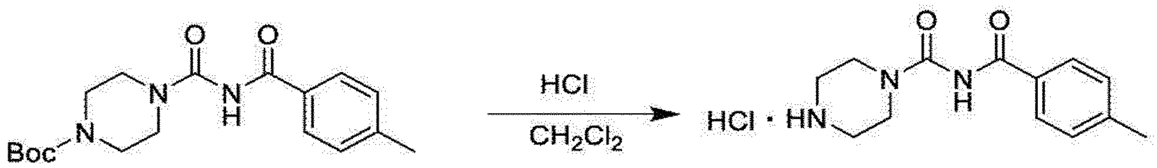


A flame dried round bottom flask equipped with a magnetic stirrer was charged with a solution of anhydrous $CH_2Cl_2$ (2 ml) and SMI (0.28 mmol). HCl (2 mmol) in diethyl ether (2.8 mmol) was added to RM at 0 °C and stirred

overnight. Then, the RM was filtered to obtain the desired product as a white solid.

Retention factor: NA
Yield: 73 mg (92%)
NMR data: 1H NMR (500 MHz, DMSO): δ10.30 (s, 1H), 9.44 (s, 2H), 7.78 (d, *J* = 8.2 Hz, 2H), 7.30-7.29 (m, 2H), 3.67-3.66 (m, 4H), 3.11 (s, 4H), 2.36 (s, 3H); 13C NMR (125 MHz, DMSO): δ 166.3, 152.7, 142.6, 130.2, 128.9, 128.2, 42.5, 21.0.

**20. Synthesis of Compound 33**

[Scheme 20]

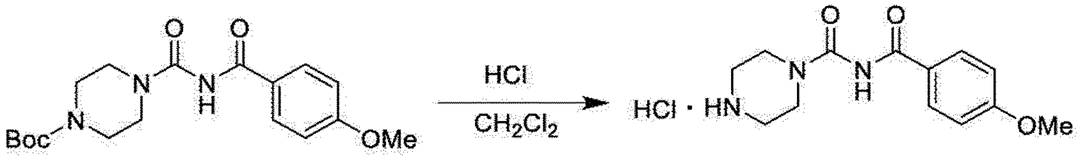


A flame dried round bottom flask equipped with a magnetic stirrer was charged with a solution of anhydrous $CH_2Cl_2$ (2 ml) and SMI (0.31 mmol). HCl (2 mmol) in diethyl ether (3.1 mmol) was added to RM at 0 °C and stirred overnight. Then, the RM was filtered to obtain the desired product as a white solid.

Retention factor: NA
Yield: 84.56 mg (91%)
NMR data: 1H NMR (500 MHz, DMSO): δ10.22 (s, 1H), 9.45 (s, 2H), 7.88 (d, *J* = 8.85 Hz, 2H), 7.02 (d, *J* = 8.9 Hz, 2H), 3.82 (s, 3H), 3.66-3.64 (m, 4H), 3.11 (s, 4H); 13C NMR (125 MHz, DMSO): δ 166.7, 162.5, 152.9, 130.3, 125.0, 113.6, 55.5, 42.5.

**21. Synthesis of Compound 36**

[Scheme 21]

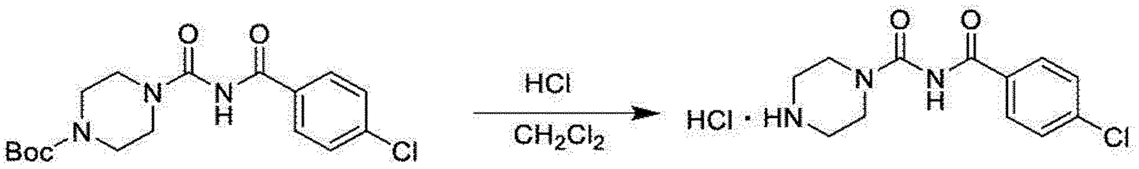


A flame dried round bottom flask equipped with a magnetic stirrer was charged with a solution of anhydrous $CH_2Cl_2$ (2 ml) and SMI (0.38 mmol). HCl (2 mmol) diethyl ether (3.8 mmol) was added to RM at 0 °C and stirred overnight. Then, the RM was filtered to obtain the desired product as a white solid.

Retention factor: NA
Yield: 106 mg (92%)
NMR data: 1H NMR (500 MHz, DMSO): δ10.50 (s, 1H), 9.50 (s, 2H), 7.89 (d, *J* = 8.86 Hz, 2H), 7.57 (d, *J* = 8.6 Hz, 2H), 3.68-3.66 (m, 4H), 3.11 (s, 4H); 13C NMR (125 MHz, DMSO): δ 165.6, 152.4, 137.1, 131.9, 130.1, 128.4, 42.5.

**22. Synthesis of Compound 43**

[Scheme 22]

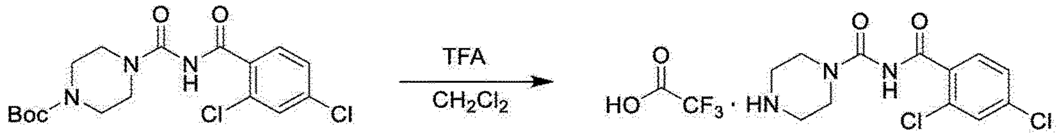


A flame dried round bottom flask equipped with a magnetic stirrer was charged with a solution of anhydrous $CH_2Cl_2$ (5 ml) and SMI (0.38 mmol). TFA (7.6 mmol) was added to RM and stirred overnight. Then, the RM was concentrated in vacuum using methanol and dichloromethane (DCM) to yield a solid product. The obtained product was washed with DCM and filtered to obtain the desired product as a white solid.

Retention factor: NA
Yield: 142 mg (90%)
NMR data: 1H NMR (500 MHz, DMSO): δ 10.73 (s, 1H), 9.04 (s, 2H), 7.63 (s, 1H), 7.48-7.43 (m, 2H), 3.58-3.56 (m, 4H), 3.08 (s, 4H).

**23. Synthesis of Compound 45**

[Scheme 23]

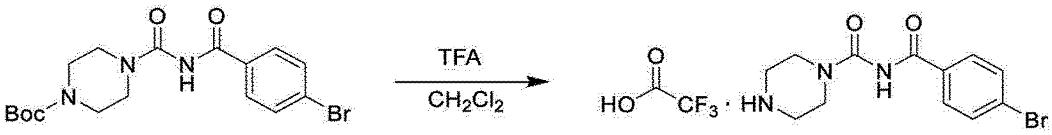


A flame dried round bottom flask equipped with a magnetic stirrer was charged with a solution of anhydrous $CH_2Cl_2$ (5 ml) and SMI (0.26 mmol). TFA (5.2 mmol) was added to RM and stirred overnight. Then, the RM was concentrated in vacuum using methanol and dichloromethane (DCM) to yield a solid product. The obtained product was washed with DCM and filtered to obtain the desired product as a white solid.

Retention factor: NA
Yield: 97.5 mg (88%)
NMR data: 1H NMR (500 MHz, DMSO): δ 10.48 (s, 1H), 9.14 (s, 2H), 7.80-7.71 (m, 4H), 3.64 (s, 4H), 3.16 (s, 4H).

**24. Synthesis of Compound 104**

[Scheme 24]

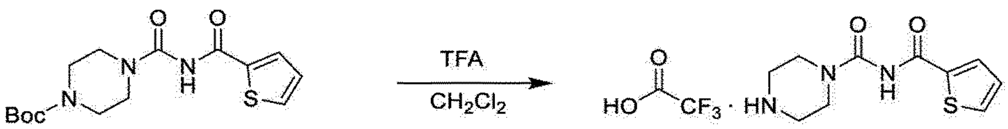


A flame dried round bottom flask equipped with a magnetic stirrer was charged with a solution of anhydrous $CH_2Cl_2$ (5 ml) and SMI (0.32 mmol). TFA (6.4 mmol) was added to RM and stirred overnight. Then, the RM was concentrated in vacuum using methanol and dichloromethane (DCM) to yield a solid product. The obtained product was washed with DCM and filtered to obtain the desired product as a white solid.

Retention factor: NA
Yield: 104 mg (92%)

NMR data: 1H NMR (500 MHz, DMSO): δ 10.42 (s, 1H), 9.04 (s, 2H), 7.94-7.86 (m, 2H), 7.19- 7.16 (m, 1H), 3.64-3.62 (m, 4H), 3.16-3.14 (m, 4H).

**25. Synthesis of Compound 129**

[Scheme 25]

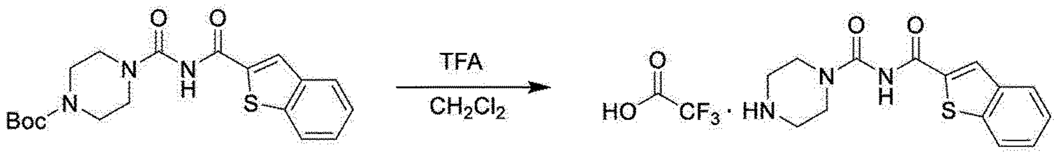


A flame dried round bottom flask equipped with a magnetic stirrer was charged with a solution of anhydrous $CH_2Cl_2$ (5 ml) and SMI (0.23 mmol). TFA (4.6 mmol) was added to RM and stirred overnight. Then, the RM was concentrated in vacuum using methanol and dichloromethane (DCM) to yield a solid product. The obtained product was washed with DCM and filtered to obtain the desired product as a white solid.

Retention factor: NA
Yield: 79 mg (85%)
NMR data: 1H NMR (500 MHz, DMSO): δ 10.68 (s, 1H), 8.97 (s, 2H), 8.31 (s, 1H), 8.07-8.05 (m, 1H), 7.08-7.98 (m, 1H), 7.53-7.42 (m, 2H), 3.67-3.65 (m, 4H), 3.17 (s, 4H).

**26. Synthesis of Compound 47**

[Scheme 26]

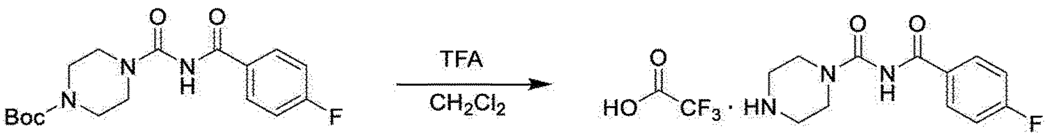


A flame dried round bottom flask equipped with a magnetic stirrer was charged with a solution of anhydrous $CH_2Cl_2$ (5 ml) and SMI (0.36 mmol). TFA (7.2 mmol) was added to RM and stirred overnight. Then, the RM was concentrated in vacuum using methanol and dichloromethane (DCM) to yield a solid product. The obtained product was washed with DCM and filtered to obtain the desired product as a white solid.

Retention factor: NA
Yield: 105 mg (80%)
NMR data: 1H NMR (500 MHz, DMSO): δ 10.42 (s, 1H), 9.08 (s, 2H), 7.96-7.94 (m, 2H), 7.35- 7.27 (m, 2H), 3.65-3.63 (m, 4H), 3.16 (s, 4H).

**27. Synthesis of Compound 154**

[Scheme 27]

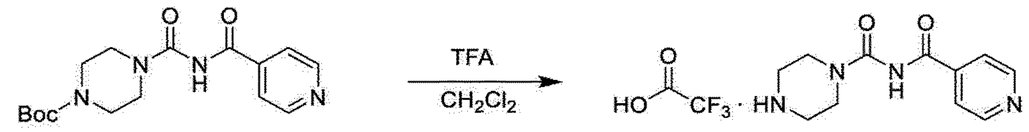


A flame dried round bottom flask equipped with a magnetic stirrer was charged with a solution of anhydrous $CH_2Cl_2$ (5 ml) and SMI (0.50 mmol). TFA (10 mmol) was added to RM and stirred about overnight. Then, the RM was concentrated in vacuum using methanol and dichloromethane (DCM) to yield a solid product. The obtained product was washed with DCM and filtered to obtain the desired product as a white solid.

Retention factor: NA
Yield: 150 mg (86.13)
NMR data: 1H NMR (500 MHz, DMSO): δ 10.82 (s, 1H), 9.31 (s, 2H), 8.80 (s, 2H), 7.82 (s, 2H), 3.67 (s, 4H), 3.17 (s, 4H).

**28. Synthesis of Compound 44**

[Scheme 28]

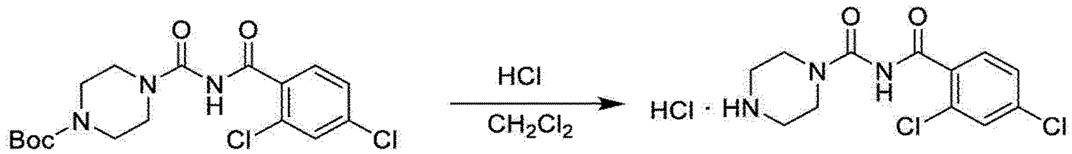


A flame dried round bottom flask equipped with a magnetic stirrer was charged with a solution of anhydrous $CH_2Cl_2$ (2 ml) and SMI (0.20 mmol). HCl (2 mmol) in diethyl ether (1 ml) was added to RM at 0 °C and stirred overnight. Then, the RM was filtered to obtain the desired product as a white solid.

Retention factor: NA
Yield: 62 mg (91.55%)
NMR data: 1H NMR (500 MHz, DMSO): δ 10.79 (s, 1H), 9.46 (s, 2H), 7.69 (d, J= 1.85 Hz, *1*H), 7.54-7.48 (m, 2H), 3.66-3.64 (m, 4H), 3.10-3.08 (m, 4H).

**29. Synthesis of Compound 46**

[Scheme 29]

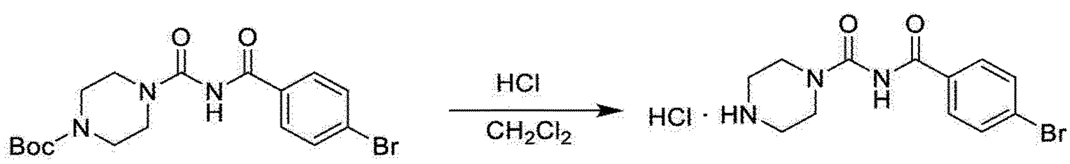


A flame dried round bottom flask equipped with a magnetic stirrer was charged with a solution of anhydrous $CH_2Cl_2$ (2 ml) and SMI (0.19 mmol). HCl (2 mmol) in diethyl ether (0.95 ml) was added to RM at 0 °C and stirred overnight. Then, the RM was filtered to obtain the desired product as a white solid.

Retention factor: NA
Yield: 61 mg (92.10%)
NMR data: 1H NMR (500 MHz, DMSO): δ 10.51 (s, 1H), 9.53 (s, 2H), 7.81 (d, *J* = 8.6 Hz, 2H), 7.70 (d, *J* = 8.6 Hz,

2H), 3.68-3.66 (m, 4H), 3.10 (s, 4H); 13C NMR (125 MHz, DMSO): δ 165.8, 152.4, 132.3, 131.4, 130.2, 126.2, 42.5.

### 30. Synthesis of Compound 130

[Scheme 30]

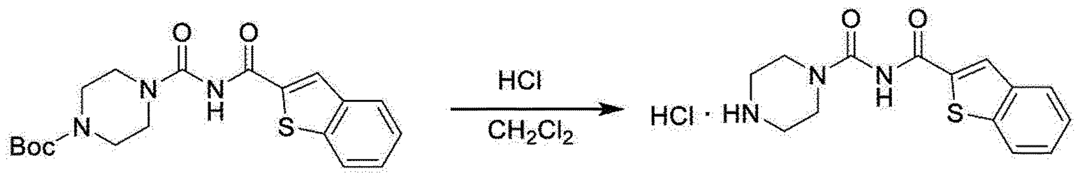


A flame dried round bottom flask equipped with a magnetic stirrer was charged with a solution of anhydrous $CH_2Cl_2$ (2 ml) and SMI (0.21 mmol). HCl (2 mmol) in diethyl ether (1.05 ml) was added to RM at 0 °C and stirred overnight. Then, the RM was filtered to obtain the desired product as a white solid.

Retention factor: NA
Yield: 60 mg (87.70%)
NMR data: 1H NMR (500 MHz, DMSO): δ 10.76 (s, 1H), 9.53 (s, 2H), 8.38 (s, 1H), 8.06- 8.04 (m, 1H), 8.00-7.97 (m, 1H), 7.52-7.45 (m, 2H), 3.72-3.69 (m, 4H), 3.15-3.13 (m, 4H).

### 31. Synthesis of Compound 155

[Scheme 31]

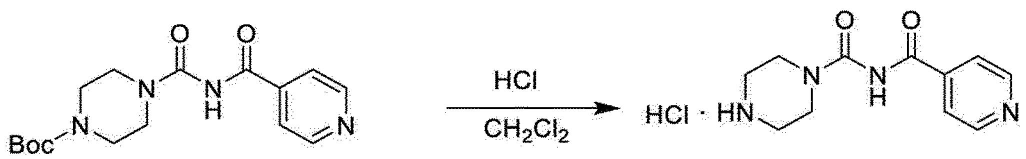


A flame dried round bottom flask equipped with a magnetic stirrer was charged with a solution of anhydrous $CH_2Cl_2$ (2 ml) and SMI (1 mmol). HCl (2 mmol) in diethyl ether (2 ml) was added to RM at 0 °C and stirred overnight. Then, the RM was filtered to obtain the desired product as a white solid.

Retention factor: NA
Yield: 97 mg (89.50%)
NMR data: 1H NMR (500 MHz, DMSO): δ 11.17 (s, 1H), 9.40 (s, 2H), 8.98- 8.95 (m, 2H), 8.16- 8.14 (m, 2H), 3.53-3.51 (m, 4H), 3.02-2.98 (m, 4H).

### 32. Synthesis of Compound 48

[Scheme 32]

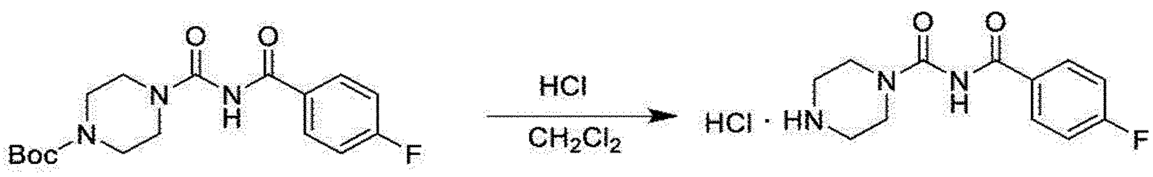

A flame-dried round bottom flask equipped with a magnetic stirrer was charged with a solution of anhydrous $CH_2Cl_2$ (2 ml) and SMI (0.31 mmol), HCl (2 mmol) in diethyl ether (3.1 mmol) was added to RM at 0 °C and stirred overnight at room temperature. Then, the RM was filtered to obtain the desired product as a white solid.

Retention factor: NA
Yield: > 90 %
NMR data: 1H NMR (500 MHz, DMSO): δ 10.45 (s, 1H), 9.51 (m, 2H), 7.97-7.95 (m, 2H), 7.35-7.32 (m, 2H), 3.69-3.66 (m, 4H), 3.12-3.10 (m, 4H).

**33. Synthesis of Compound 105**

[Scheme 33]

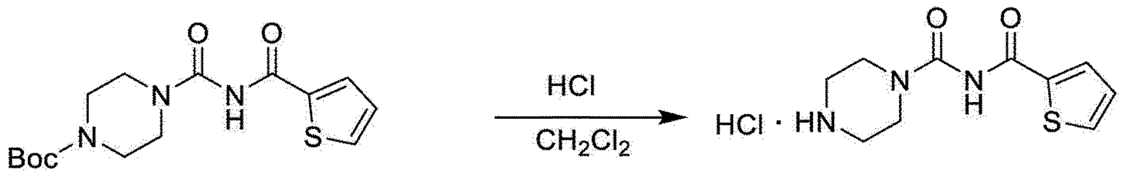


A flame-dried round bottom flask equipped with a magnetic stirrer was charged with a solution of anhydrous $CH_2Cl_2$ (2 ml) and SMI (0.35 mmol), and HCl (2 mmol) in diethyl ether (3.5 mmol) was added to RM at 0 °C and stirred overnight at room temperature. Then, the RM was filtered to obtain the desired product as a white solid.

Retention factor: NA
Yield: > 90 %
NMR data: 1H NMR (500 MHz, DMSO): δ 10.46 (s, 1H), 9.35 (s, 2H), 8.02-8.01 (m, 1H), 7.92-7.91 (m, 1H), 7.21 (dd, *J* = 5, 3.8 Hz, 1H), 3.69-3.66 (m, 4H), 3.12-3.10 (m, 4H).

**34. Synthesis of Compound 51**

[Scheme 34]

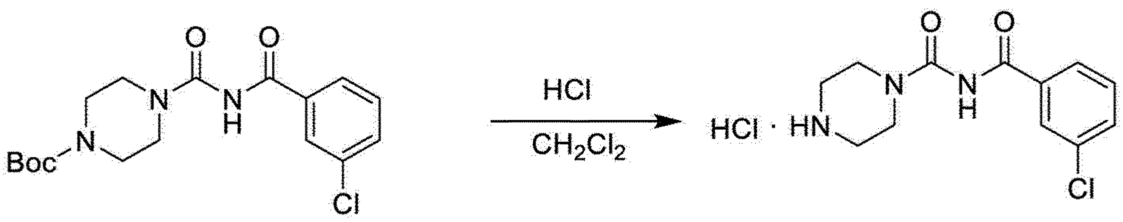


A flame-dried round bottom flask equipped with a magnetic stirrer was charged with a solution of anhydrous $CH_2Cl_2$ (2 ml) and SMI (0.30 mmol), HCl (2 mmol) in diethyl ether (3.0 mmol) was added to RM at 0 °C and stirred overnight at room temperature. Then, the RM was filtered to obtain the desired product as a white solid.

Retention factor: NA
Yield: > 90 %
NMR data: 1H NMR (500 MHz, DMSO): δ 10.54 (s, 1H), 9.52 (s, 2H), 7.92-7.91 (m, 1H), 7.84-7.82 (m, 1H), 7.68-7.65 (m, 1H) 7.55-7.52 (m, 1H), 3.69-3.67 (m, 4H), 3.10 (s, 4H).

**35. Synthesis of Compound 53**

[Scheme 35]

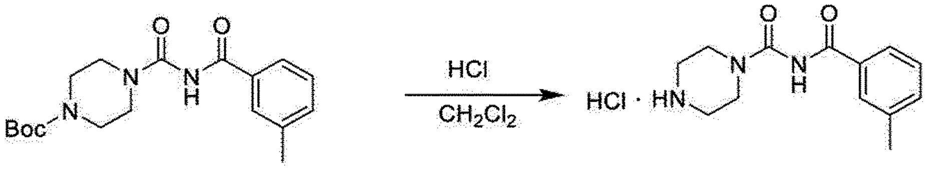


A flame-dried round bottom flask equipped with a magnetic stirrer was charged with a solution of anhydrous $CH_2Cl_2$ (2 ml) and SMI (0.30 mmol), HCl (2 mmol) in diethyl ether (3.0 mmol) was added to RM at 0 °C and stirred overnight at room temperature. Then, the RM was filtered to obtain the desired product as a white solid.

Retention factor: NA
Yield: > 85 %
NMR data: 1H NMR (500 MHz, DMSO): δ 10.39 (s, 1H), 9.66 (s, 2H), 7.74-7.69 (m, 2H), 7.43-7.37 (m, 2H), 3.71-3.69 (m, 4H), 3.17-3.13 (m, 4H), 2.37 (s, 3H).

**36. Synthesis of Compound 55**

[Scheme 36]

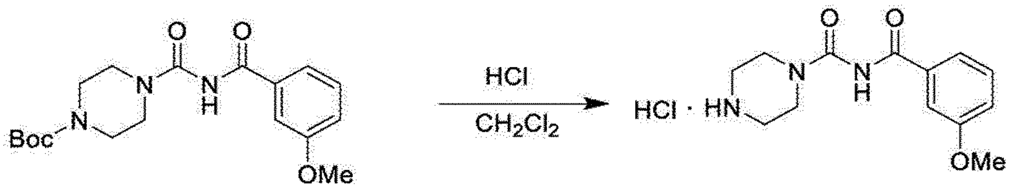


A flame-dried round bottom flask equipped with a magnetic stirrer was charged with a solution of anhydrous $CH_2Cl_2$ (2 ml) and SMI (0.37 mmol), and HCl (2 mmol) in diethyl ether (3.7 mmol) was added to RM at 0 °C and stirred overnight at room temperature. Then, the RM was filtered to obtain the desired product as a white solid.

Retention factor: NA
Yield: > 85 %
NMR data: 1H NMR (500 MHz, DMSO): δ 10.43 (s, 1H), 9.65 (s, 2H), 7.48-7.39 (m, 3H), 7.17-7.15 (m, 1H), 3.80 (s, 3H), 3.69-3.67 (m, 4H), 3.12-3.09 (m, 4H).

**37. Synthesis of Compound 52**

[Scheme 37]

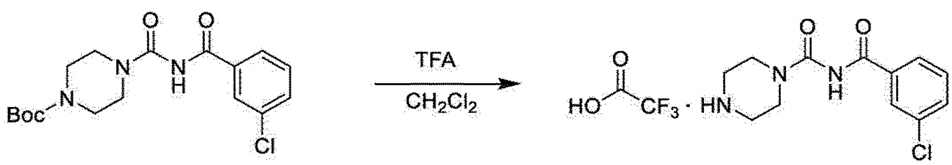


A flame dried round bottom flask equipped with a magnetic stirrer was charged with a solution of anhydrous $CH_2Cl_2$ (2 ml) and SMI (0.43 mmol). TFA (8.6 mmol) was added to RM at 0 °C and stirred overnight at room temperature. Then, the RM was concentrated in vacuum using methanol and dichloromethane (DCM) to obtain a solid product. The obtained product was washed with DCM and filtered to obtain the desired product as a white solid.

Retention factor: NA
Yield: >90%
NMR data: 1H NMR (500 MHz, DMSO): δ 10.52 (s, 1H), 9.19 (s, 2H), 7.90-7.89 (m, 1H), 7.83-7.81 (m, 1H), 7.68-7.66 (m, 1H) 7.56-7.52 (m, 1H), 3.66-3.64 (m, 4H), 3.17-3.16 (m, 4H).

**38. Synthesis of Compound 54**

[Scheme 38]

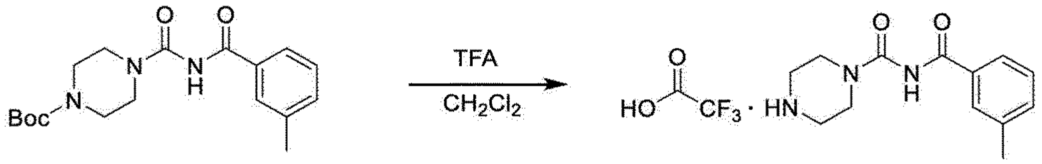


A flame dried round bottom flask equipped with a magnetic stirrer was charged with a solution of anhydrous $CH_2Cl_2$ (2 ml) and SMI (0.40 mmol). TFA (8 mmol) was added to RM at 0 °C and stirred overnight at room temperature. Then, the RM was concentrated in vacuum using methanol and dichloromethane (DCM) to obtain a solid product. The obtained product was washed with DCM and filtered to obtain the desired product as a white solid.

Retention factor: NA
Yield: > 85 %
NMR data: 1H NMR (500 MHz, DMSO): δ 10.37 (s, 1H), 9.31 (s, 2H), 7.70-7.65 (m, 2H), 7.40-7.35 (m, 2H), 3.66-3.64 (m, 4H), 3.16-3.14 (m, 4H), 2.35 (s, 3H).

**39. Synthesis of Compound 56**

[Scheme 39]

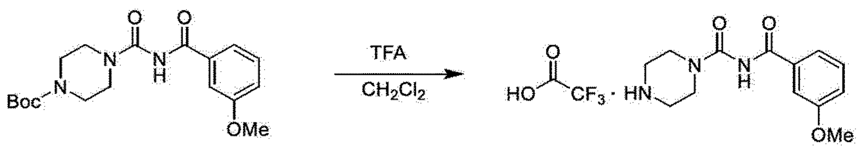


A flame dried round bottom flask equipped with a magnetic stirrer was charged with a solution of anhydrous $CH_2Cl_2$ (2 ml) and SMI (0.39 mmol). TFA (7.8 mmol) was added to RM at 0 °C and stirred overnight at room temperature. Then, the RM was concentrated in vacuum using methanol and dichloromethane (DCM) to obtain a solid product. The obtained product was washed with DCM and filtered to obtain the desired product as a white solid.

Retention factor: NA
Yield: > 85%
NMR data: 1H NMR (500 MHz, DMSO): δ 10.39 (s, 1H), 9.23 (s, 2H), 7.47-7.39 (m, 3H), 7.17-7.15 (m, 1H), 3.80 (s, 3H), 3.65-3.63 (m, 4H), 3.16-3.14 (m, 4H).

## Example

### 1. Confirmation of agmatinase inhibitory activity of the compound - using Urea Assay

#### (1) Experimental reagents and manufacturing methods

The agmatinase inhibitory activity (FIG. 1) was measured for some of the compounds of the present invention shown in Table 1.

Mg containing buffer was used as the assay buffer, and agmatinase (speB) and recombinant protein were used at 0.1 ml (final concentration: 2 mg/ml

#### (2) Experimental method and result

After adding the sample and agmatinase to the activity buffer and performing a reaction, 1 ul (final conc. 500 uM) of agmatine was added. Thereafter, the assay was performed to measure the absorbance, and the amount was converted according to the standard curve.

Calculation of the urea concentration was calculated using Equation 1 below.

[Equation 1]

$$[Urea] = \frac{OD_{SAMPLE} - OD_{BLANK}}{OD_{STANDARD} - OD_{BLANK}} \times n \times [STD] \ (mg/dL)$$

After treatment at concentrations of 1 uM, 10 uM, 30 uM, 50 uM and 300 uM for each compound, the agmatinase inhibitory activity was determined in percent. As shown in FIG. 1, it can be seen that the higher the concentration, the larger the agmatinase inhibitory activity.

As shown in FIG. 1, it can be seen that most of the compounds of the present invention exhibited agmatinase inhibitory activity.

### 2. Confirmation of sociability and cognitive improvement effects of compounds in autism animal models

In order to confirm therapeutic effects of the compound according to the present invention on lack of sociability, which is one of the phenotypes of developmental disorders, a three-chamber test was performed using a valproic acid (VPA)-induced autism animal model. The chamber duration is shown as stranger (retention time to mouse), empty (retention time to empty space), and center (retention time to center space), and if the mouse stay time is longer than empty space, it is determined to be social.

As a result of the experiment, in the case of VPA mice, the retention time to empty space was longer than the retention time to mouse, which means that sociality was reduced.

On the other hand, when VPA mice were treated with the compound of the present invention, it was confirmed that the retention time to mouse was higher than the retention time to empty space, which means that the sociality of the VPA mice was improved by the compound treatment (FIG. 2).

A stimulus mouse (familiar) was explored for 10 minutes in the experimental animals, a new stimulus mouse (novel) was added to the experimental animals. Then, after 10 minutes of exploration again, the result of evaluating whether or not the novel mouse spent more time than the familiar mouse was confirmed (FIG. 3). At this time, the old mouse is called familiar, and the newly introduced mouse is called novel (if a spending time with the novel mouse is longer than that of the familiar mouse, it is determined that there is a social cognitive ability). In other words, it is determined that it has the cognitive ability to distinguish between the existing familiar mouse and the new novel mouse.

The experimental results were expressed by measuring a time for the test mice to explore each mouse or a sniffing time for each mouse of the test mice. It means that the more time spent with the novel mouse than the familiar mouse or the more time spent sniffing the novel mouse than the familiar mouse, the higher the social preference.

As a result of the experiment, in the case of VPA mice, they spent more time with familiar mice than with novel mice, and spent more time sniffing with familiar mice than with novel mice, which means that their social preference was reduced.

On the other hand, when VPA mice were treated with the compound of the present invention, more time was spent with novel mice than familiar mice, and sniffing time was increased for novel mice than for familiar mice. Through this, it can be seen that treatment with the compound of the present invention can improve social preference (FIGS. 2 to 3).

Through the results of confirming sociability and social preference, when sociability and social preference are improved, symptoms of sociability and social cognitive intelligence are improved, and when these symptoms are improved, there will be effects of preventing or treating not only emotional and behavioral disorders such as autism spectrum disorder and schizophrenia, but also cognitive related intellectual disability or degenerative neurological diseases such as Alzheimer's disease and Parkinson's diseases, such that it may be predicted that administering the compound of the present invention to a patient group with emotional and behavioral disorder can achieve preventive or therapeutic effects.

**Repetitive behavior experiment**

In order to confirm the therapeutic effect of the compound according to the present invention on homology, one of the phenotypes of developmental disorders, a self-grooming test was performed using a valproic acid (VPA)-induced autism animal model (FIG. 4).

The animals were placed in an empty cage, and the duration of licking or grooming (specified as self-grooming) per hour was measured and graphed. If the duration is increased, it means that repetitive behavior appears.

Through the results of demonstrating repetitive behavior, if the repetitive behavior is improved, the symptoms of stereotyped behavior and impulsivity may be improved. When the symptoms of stereotyped behavior are improved, the effect of preventing or treating emotional and behavioral disorders such as autism spectrum disorder and obsessive-compulsive disorder may be expected. Further, if the symptoms of impulsivity are improved, there will be a preventive or therapeutic effect on emotional and behavioral disorders such as autism spectrum disorder, obsessive-compulsive disorder, depression, anxiety disorder, panic disorder, and attention deficit hyperactivity disorder. Therefore, it can be predicted that administering the compound to a patient group with the emotional and behavioral disorder will attain preventive or therapeutic effects.

**GSH assay**

In order to confirm the neuroprotective effect of the compound according to the present invention, after 24 hours of pretreatment, cell oxidation stimulation was induced with $H_2O_2$, and after performing the GSH assay (FIG. 5), cell images were confirmed (FIG. 6). The cells used herein were primary nerve cells, and were treated with 0.1, 1, 10, 50, 100, and 300 μM of the compound of the present invention.

## Claims

1. A compound represented by Formula 1 below, a stereoisomer or a pharmaceutically acceptable salt thereof:

[Formula 1]

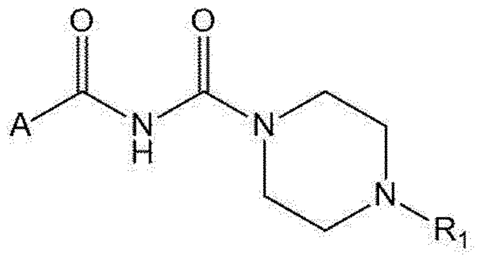

(wherein, $R_1$ is hydrogen or a tert-butyloxycarbonyl group,
A is a single or double cyclic group of $C_5$-$C_{10}$,
each ring of the cyclic group is unsubstituted or substituted with 1 to 3 heteroatoms, and
the cyclic group is unsubstituted or substituted with halogen, $C_1$-$C_5$ alkyl, $C_1$- $C_5$ alkoxy, CN or $NO_2$).

2. The compound, a stereoisomer or a pharmaceutically acceptable salt thereof according to claim 1, wherein A is selected from the group consisting of the following cyclic groups:

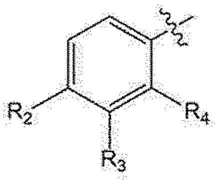 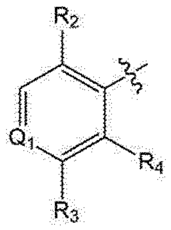 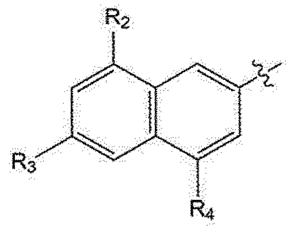

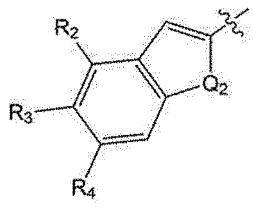 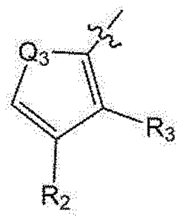

(wherein $R_2$, $R_3$ and $R_4$ are each independently hydrogen, halogen, $C_1$-$C_5$ alkyl, $C_1$-$C_5$ alkoxy, CN or $NO_2$, and $Q_1$, $Q_2$ and $Q_3$ are each independently N, O or S).

3. The compound, a stereoisomer or a pharmaceutically acceptable salt thereof according to claim 1, wherein A is selected from the group consisting of the following cyclic groups:

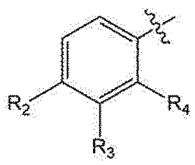 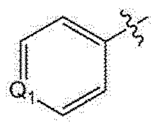 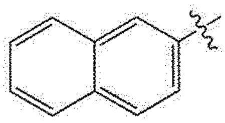

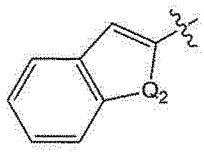 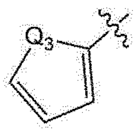

(wherein $R_2$, $R_3$ and $R_4$ are each independently hydrogen, halogen, $C_1$-$C_5$ alkyl, $C_1$-$C_5$ alkoxy, CN or $NO_2$; and $Q_1$, $Q_2$ and $Q_3$ are each independently N, O or S).

4. The compound, a stereoisomer or a pharmaceutically acceptable salt thereof according to claim 1, wherein A is selected from the group consisting of the following cyclic groups:

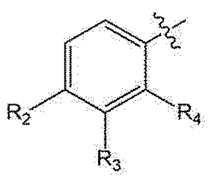 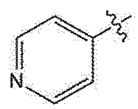 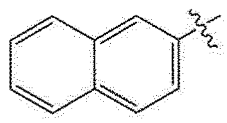

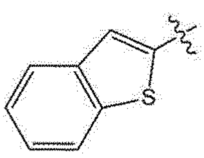 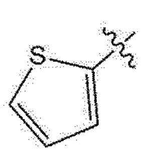 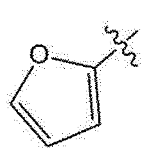

(wherein $R_2$, $R_3$ and $R_4$ are each independently hydrogen, halogen, $C_1$-$C_5$ alkyl, $C_1$-$C_5$ alkoxy, CN or $NO_2$).

5. The compound, a stereoisomer or a pharmaceutically acceptable salt thereof according to claim 1, wherein the

compound is selected from the group consisting of the following compounds:

tert-butyl-4-((4-methylbenzoyl)carbamoyl)piperazine-1-carboxylate;
tert-butyl-4-((4-methoxybenzoyl)carbamoyl)piperazine-1-carboxylate;
tert-butyl-4-((4-chlorob enzoyl)carb amoyl)piperazine-1-carb oxylate;
tert-butyl-4-(b enzoylcarb amoyl)piperazine-1-carboxylate;
N-(4-methylbenzoyl)piperazine-1-carboxamide;
N-(4-methylbenzoyl)piperazine-1-carboxamide-2,2,2-trifluoroacetate;
N-(4-methoxybenzoyl)piperazine-1-carboxamide-2,2,2-trifluoroacetate;
N-(4-chlorobenzoyl)piperazine-1-carboxamide-2,2,2-trifluoroacetate;
N-benzoylpiperazine-1-carboxamide-2,2,2-tri fluoroacetate;
N-(2-naphthoyl)piperazine-1-carboxamide-2,2,2-trifluoroacetate;
N-(4-cyanobenzoyl)piperazine-1-carboxamide-2,2,2-trifluoroacetate;
N-(furan-2-carbonyl)piperazine-1-carboxamide-2,2,2-trifluoroacetate;
N-(4-nitrobenzoyl)piperazine-1-carboxamide-2,2,2-trifluoroacetate;
N-(2-naphthoyl)piperazine-1-carboxamide hydrochloride;
N-(4-cyanobenzoyl)piperazine-1-carboxamide hydrochloride;
N-(4-nitrobenzoyl)piperazine-1-carboxamide hydrochloride;
N-benzoylpiperazine-1-carboxamide hydrochloride;
N-(furan-2-carbonyl)piperazine-1-carboxamide hydrochloride;
N-(4-methylbenzoyl)piperazine-1-carboxamide hydrochloride;
N-(4-methoxybenzoyl)piperazine-1-carboxamide hydrochloride;
N-(4-chlorobenzoyl)piperazine-1-carboxamide hydrochloride;
N-(2,4-dichlorobenzoyl)piperazine-1-carboxamide-2,2,2-trifluoroacetate;
N-(4-bromobenzoyl)piperazine-1-carboxamide-2,2,2-trifluoroacetate;
N-(thiophene-2-carbonyl)piperazine-1-carbxamide-2,2,2-trifluoroacetate;
N-(benzo[b]thiophene-2-carbonyl)piperazine-1-carboxamide-2,2,2-trifluoroacetate;
N-(4-fluorobenzoyl)piperazine-1-carboxamide-2,2,2-trifluoroacetate;
N-i sonicotinoylpiperazine-1-carboxamide-2,2,2-trifluoroacetate;
N-(2,4-dichlorobenzoyl)piperazine-1-carboxamide hydrochloride;
N-(4-bromobenzoyl)piperazine-1-carboxamide hydrochloride;
N-(benzo[b]thiophene-2-carbonyl)piperazine-1-carboxamide hydrochloride;
N-isonicotinoylpiperazine-1-carboxamide hydrochloride;
N-(4-fluorobenzoyl)piperazine-1-carboxamide hydrochloride;
N-(thiophene-2-carbonyl)piperazine-1-carboxamide hydrochloride;
N-(3-chlorobenzoyl)piperazine-1-carboxamide hydrochloride;
N-(3-methylbenzoyl)piperazine-1-carboxamide hydrochloride;
N-(3-methoxybenzoyl)piperazine-1-carboxamide hydrochloride;
N-(3-chlorobenzoyl)piperazine-1-carboxamide-2,2,2-trifluoroacetate;
N-(3-methylbenzoyl)piperazine-1-carboxamide-2,2,2-trifluoroacetate; and
N-(3-methoxybenzoyl)piperazine-1-carboxamide-2,2,2-trifluoroacetate.

**6.** A pharmaceutical composition for preventing or treating emotional and behavioral disorder-related symptoms, the pharmaceutical composition comprising the compound, a stereoisomer or a pharmaceutically acceptable salt thereof as defined in any one of claims 1 to 5.

**7.** The pharmaceutical composition, wherein claim 6, wherein the emotional and behavioral disorder-related symptoms are any one selected from the group consisting of lack of sociability, lack of social cognitive intelligence, stereotyped behavior, hyperactivity, impulsivity and distraction.

**8.** A pharmaceutical composition for preventing or treating emotional and behavioral disorders, the pharmaceutical composition comprising the compound, a stereoisomer or a pharmaceutically acceptable salt thereof as defined in any one of claims 1 to 5.

**9.** A pharmaceutical composition for preventing or treating degenerative neurological diseases, the pharmaceutical composition comprising the compound, a stereoisomer or a pharmaceutically acceptable salt thereof as defined in any one of claims 1 to 5.

**10.** The pharmaceutical composition according to claim 8, wherein the emotional and behavioral disorder is any one selected from the group consisting of autism spectrum disorder, schizophrenia, obsessive-compulsive disorder, depression, anxiety disorder, panic disorder and attention deficit hyperactivity disorder.

[FIG. 1]

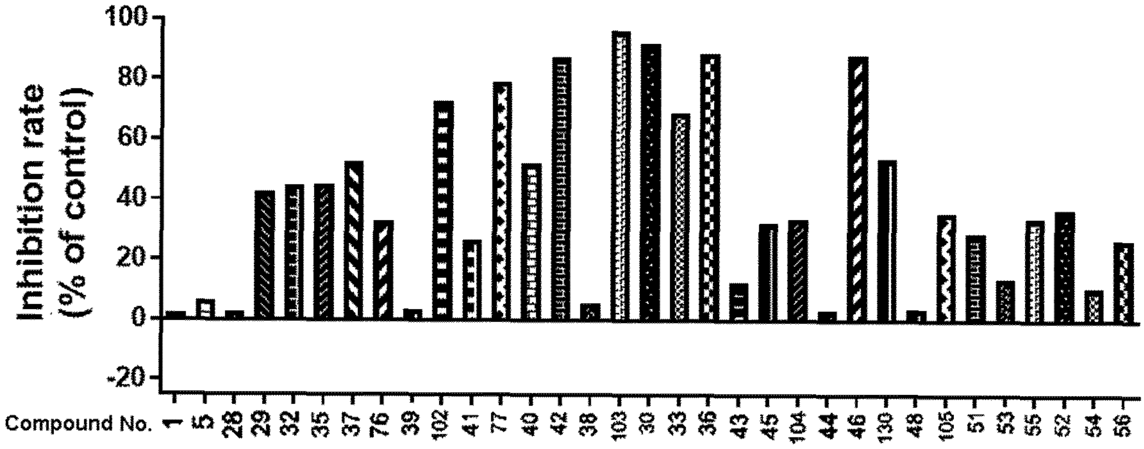
Inhibition rate
(% of control)
100
80
60
40
20
0
-20
Compound No. 1 5 28 29 32 35 37 76 39 102 41 77 40 42 38 103 30 33 36 43 45 104 44 46 130 48 105 51 53 55 52 54 56

[FIG. 2]

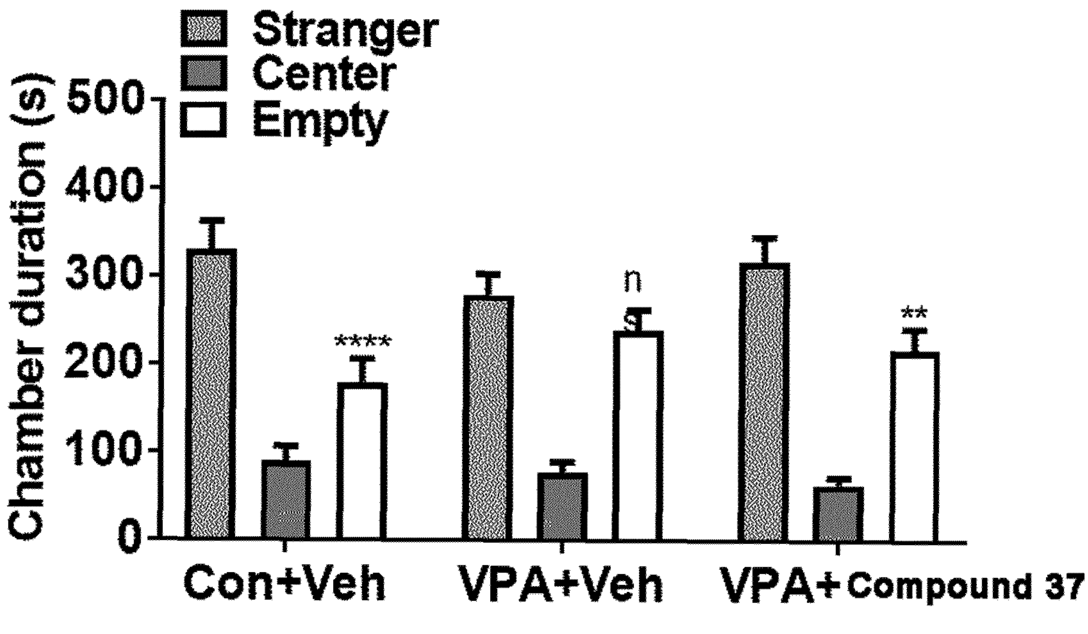
Stranger
Center
Empty
Chamber duration (s)
500
400
300
200
100
0
****
ns
**
Con+Veh
VPA+Veh
VPA+Compound 37

[FIG. 3]

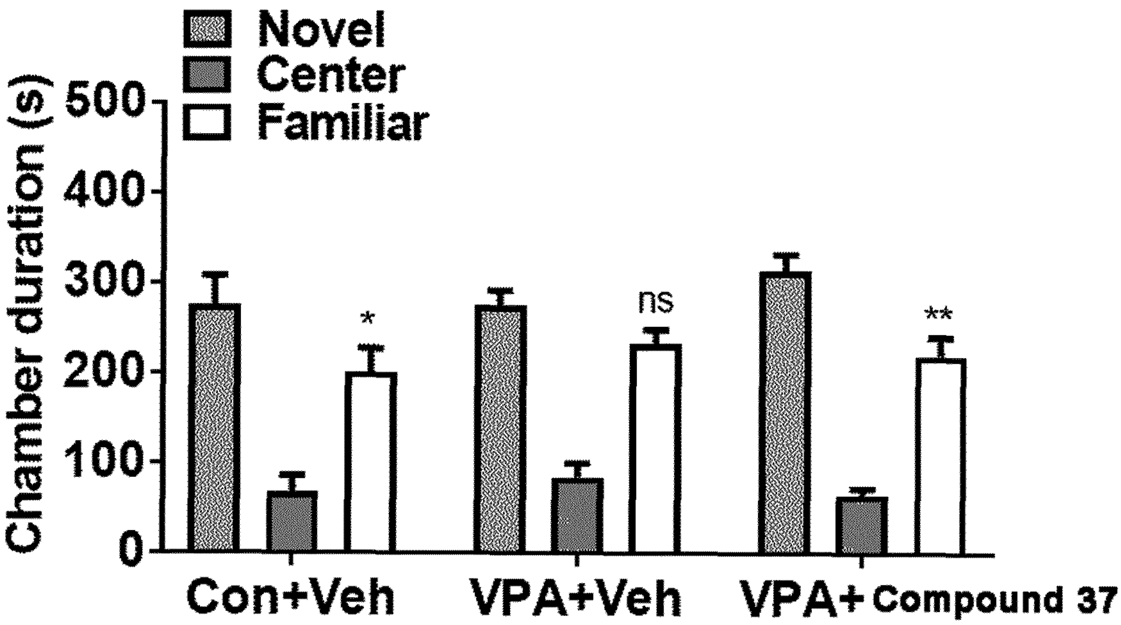
Novel
Center
Familiar
Chamber duration (s)
500
400
300
200
100
0
*
ns
**
Con+Veh
VPA+Veh
VPA+Compound 37

[FIG. 4]

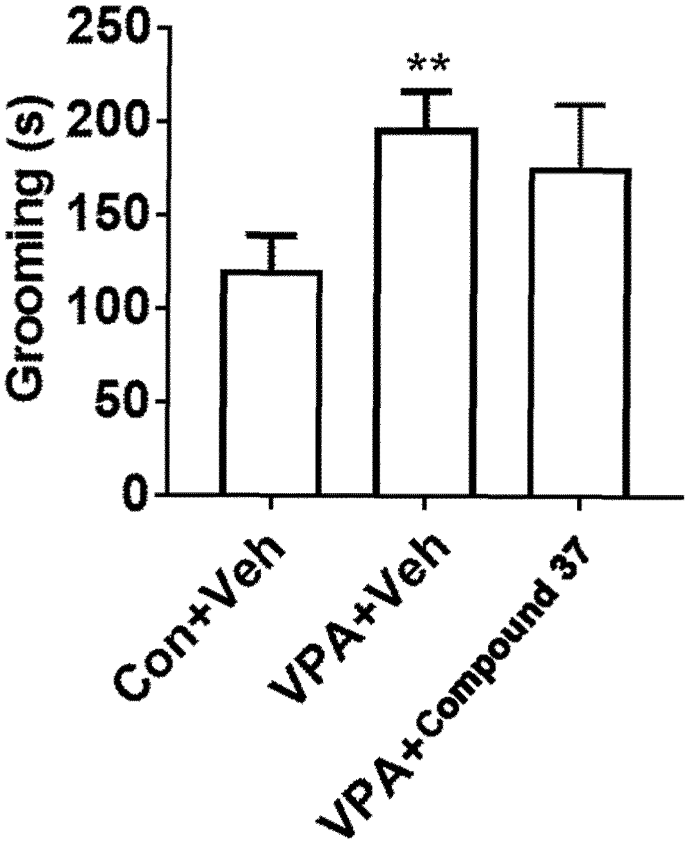
Grooming (s)
250
200
150
100
50
0
**
Con+Veh
VPA+Veh
VPA+Compound 37

[FIG. 5]

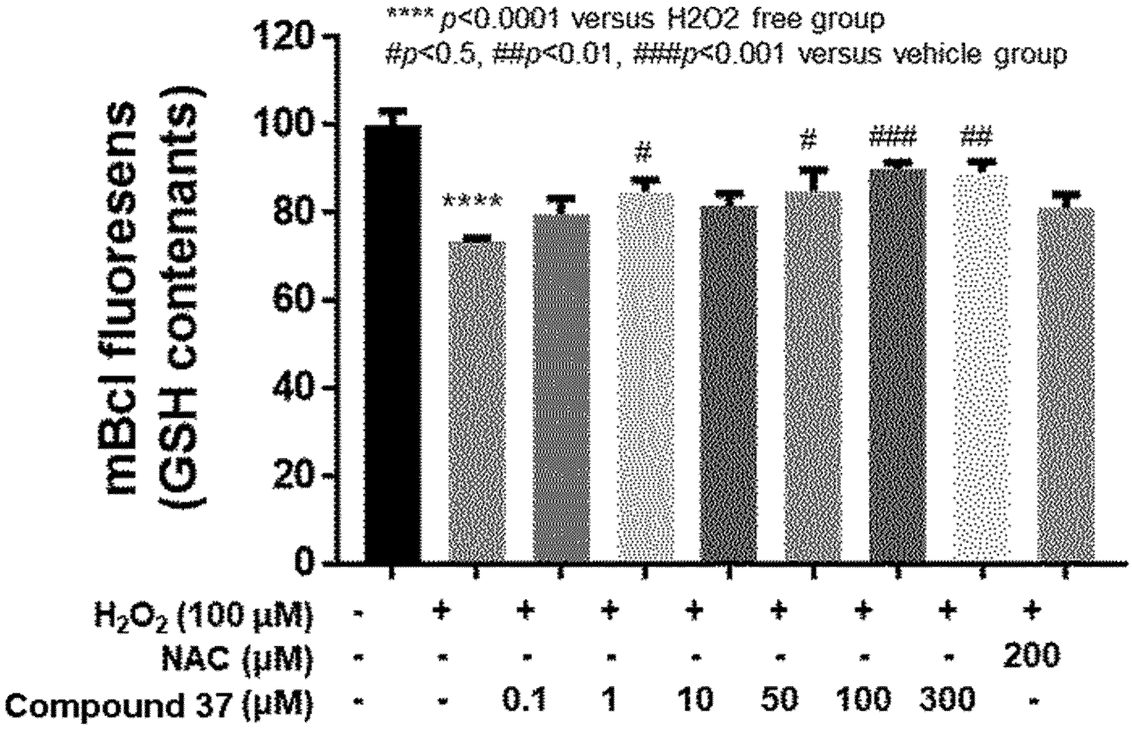
**** p<0.0001 versus H2O2 free group
#p<0.5, ##p<0.01, ###p<0.001 versus vehicle group
mBcl fluoresens (GSH contenants)
120
100
80
60
40
20
0
****
#
#
###
##
H2O2 (100 µM) - + + + + + + + +
NAC (µM) - - - - - - - - 200
Compound 37 (µM) - - 0.1 1 10 50 100 300 -

[FIG. 6]

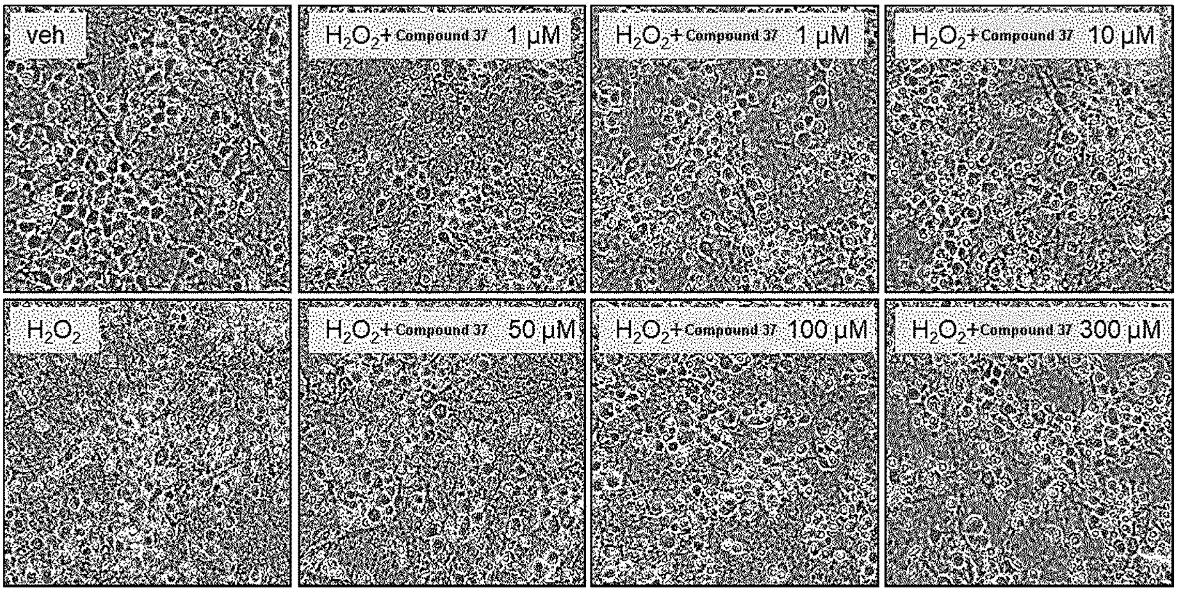
veh
H2O2+Compound 37 1 µM
H2O2+Compound 37 1 µM
H2O2+Compound 37 10 µM
H2O2
H2O2+Compound 37 50 µM
H2O2+Compound 37 100 µM
H2O2+Compound 37 300 µM

**INTERNATIONAL SEARCH REPORT**

International application No.
**PCT/KR2022/013519**

**A. CLASSIFICATION OF SUBJECT MATTER**

**C07D 295/145**(2006.01)i; **A61K 31/495**(2006.01)i; **A61K 31/496**(2006.01)i; **A61K 31/444**(2006.01)i; **A61P 25/00**(2006.01)i; **A61P 25/28**(2006.01)i; **C07D 307/68**(2006.01)i; **C07D 333/38**(2006.01)i; **C07D 213/81**(2006.01)i; **A61P 25/18**(2006.01)i

According to International Patent Classification (IPC) or to both national classification and IPC

**B. FIELDS SEARCHED**

Minimum documentation searched (classification system followed by classification symbols)

C07D 295/145(2006.01); C07D 213/82(2006.01); C07D 233/90(2006.01); C07D 239/42(2006.01); C07D 295/185(2006.01); C07D 413/04(2006.01); C07D 413/14(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, CasReact, Caplus), PubChem, Google & keywords: 피페라진(piperazine), 카복사미드(carboxamide), 정서행동장애(emotional and behavior disorder), 퇴행성신경질환(neurodegenerative disease)

**C. DOCUMENTS CONSIDERED TO BE RELEVANT**

| Category* | Citation of document, with indication, where appropriate, of the relevant passages | Relevant to claim No. |
|---|---|---|
| X<br>A | Chemical Abstract Compound. STNext. RN 1384265-94-4 (25 July 2012).<br>See the compounds. | 1-4<br>5-10 |
| A | CN 102532062 A (SHANGHAI UNIVERSITY OF ENGINEERING SCIENCE) 04 July 2012 (2012-07-04)<br>See entire document. | 1-10 |
| A | KR 10-2006-0108649 A (BAYER CROPSCIENCE GMBH) 18 October 2006 (2006-10-18)<br>See entire document. | 1-10 |
| A | LI, P. et al. A facile and efficient synthesis of 4H-1,3-benzoxazine-4-one with electron-withdrawing group derivatives. Tetrahedron letters. 2015, vol. 56, no. 32, pp. 4683-4685.<br>See entire document. | 1-10 |
| A | US 2020-0010462 A1 (YUMANITY THERAPEUTICS, INC.) 09 January 2020 (2020-01-09)<br>See entire document. | 1-10 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 December 2022** | **13 December 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**
**Information on patent family members**

International application No.
**PCT/KR2022/013519**

| Patent document cited in search report | Publication date (day/month/year) | Patent family member(s) | Publication date (day/month/year) |
|---|---|---|---|
| CN 102532062 A | 04 July 2012 | CN 102532062 B | 04 December 2013 |
| KR 10-2006-0108649 A | 18 October 2006 | BR PI0415223 A | 05 December 2006 |
| | | CN 1886381 A | 27 December 2006 |
| | | DE 10346245 A1 | 28 April 2005 |
| | | EP 1673350 A2 | 28 June 2006 |
| | | JP 2007-507442 A | 29 March 2007 |
| | | MX PA06003727 A | 23 June 2006 |
| | | TW 200530182 A | 16 September 2005 |
| | | WO 2005-035508 A2 | 21 April 2005 |
| | | WO 2005-035508 A3 | 09 June 2005 |
| US 2020-0010462 A1 | 09 January 2020 | CA 3083000 A1 | 02 May 2019 |
| | | CN 110099898 A | 06 August 2019 |
| | | EP 3529245 A1 | 28 August 2019 |
| | | EP 3529245 A4 | 23 December 2020 |
| | | EP 3700934 A1 | 02 September 2020 |
| | | EP 3700934 A4 | 27 October 2021 |
| | | JP 2019-533022 A | 14 November 2019 |
| | | MA 46589 A | 28 August 2019 |
| | | WO 2018-081167 A1 | 03 May 2018 |
| | | WO 2019-018795 A1 | 24 January 2019 |
| | | WO 2019-084157 A1 | 02 May 2019 |
| | | WO 2019-209948 A1 | 31 October 2019 |
| | | WO 2019-209948 A8 | 28 November 2019 |
| | | WO 2019-209948 A9 | 02 January 2020 |

Form PCT/ISA/210 (patent family annex) (July 2022)